(19) Europäisches Patentamt
European Patent Office
Office européen des brevets

(11) EP 4 640 238 A1

(12) EUROPEAN PATENT APPLICATION
published in accordance with Art. 153(4) EPC

(43) Date of publication:
29.10.2025 Bulletin 2025/44

(21) Application number: 23907079.0

(22) Date of filing: 20.12.2023

(51) International Patent Classification (IPC):
*A61K 45/00* (2006.01)

(52) Cooperative Patent Classification (CPC):
**A01K 67/0278; A61K 31/085; A61K 31/136; A61K 31/165; A61K 31/166; A61K 31/351; A61K 31/352; A61K 31/403; A61K 31/409; A61K 31/4439; A61K 31/444; A61K 31/453; A61K 31/454; A61K 31/4709; A61K 31/473;**
(Cont.)

(86) International application number:
PCT/JP2023/045647

(87) International publication number:
WO 2024/135719 (27.06.2024 Gazette 2024/26)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 22.12.2022 JP 2022205225

(71) Applicant: **Kyoto University**
**Kyoto-shi, Kyoto 606-8501 (JP)**

(72) Inventors:
- **KONDOH Hiroshi**
  **Kyoto-shi, Kyoto 606-8501 (JP)**
- **MIKAWA Takumi**
  **Kyoto-shi, Kyoto 606-8501 (JP)**
- **SHIBATA Eri**
  **Kyoto-shi, Kyoto 606-8501 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) THERAPEUTIC AGENT FOR PULMONARY DISEASE, HEPATIC DISEASE OR RENAL DISEASE, WHICH CONTAINS PGAM-CHK1 BINDING INHIBITOR

(57) The purpose of the present invention is to provide a novel therapeutic agent which exhibits an excellent therapeutic effect on a pulmonary disease, a hepatic disease or a renal disease, particularly pulmonary fibrosis or nephrosclerosis. The present invention is a therapeutic agent for a pulmonary disease, a hepatic disease or a renal disease, which contains an inhibitor of the binding between PGAM and Chk1 as an active ingredient. The inhibitor of the binding between PGAM and Chk1 preferably comprises at least one component selected from the group consisting of an RSK1 kinase inhibitor, an Fak kinase inhibitor, an inhibitor of a signaling pathway in which an Fak kinase is involved, a CDK inhibitor, a calcium antagonist, a cardiac glycoside, a DNA-damaging agent, an antibacterial agent, an aurora kinase inhibitor, a flavonoid, a PI3K kinase inhibitor, an HDAC inhibitor, a therapeutic agent for age-related macular degeneration, a p38MAPK inhibitor, an mTOR inhibitor, a tyrosine kinase inhibitor, a Bcl-2 inhibitor, an HIF-2$\alpha$ inhibitor, a statin, a serotonin receptor antagonist, other kinase inhibitors, and natrin3b.

EP 4 640 238 A1

[Fig. 21]

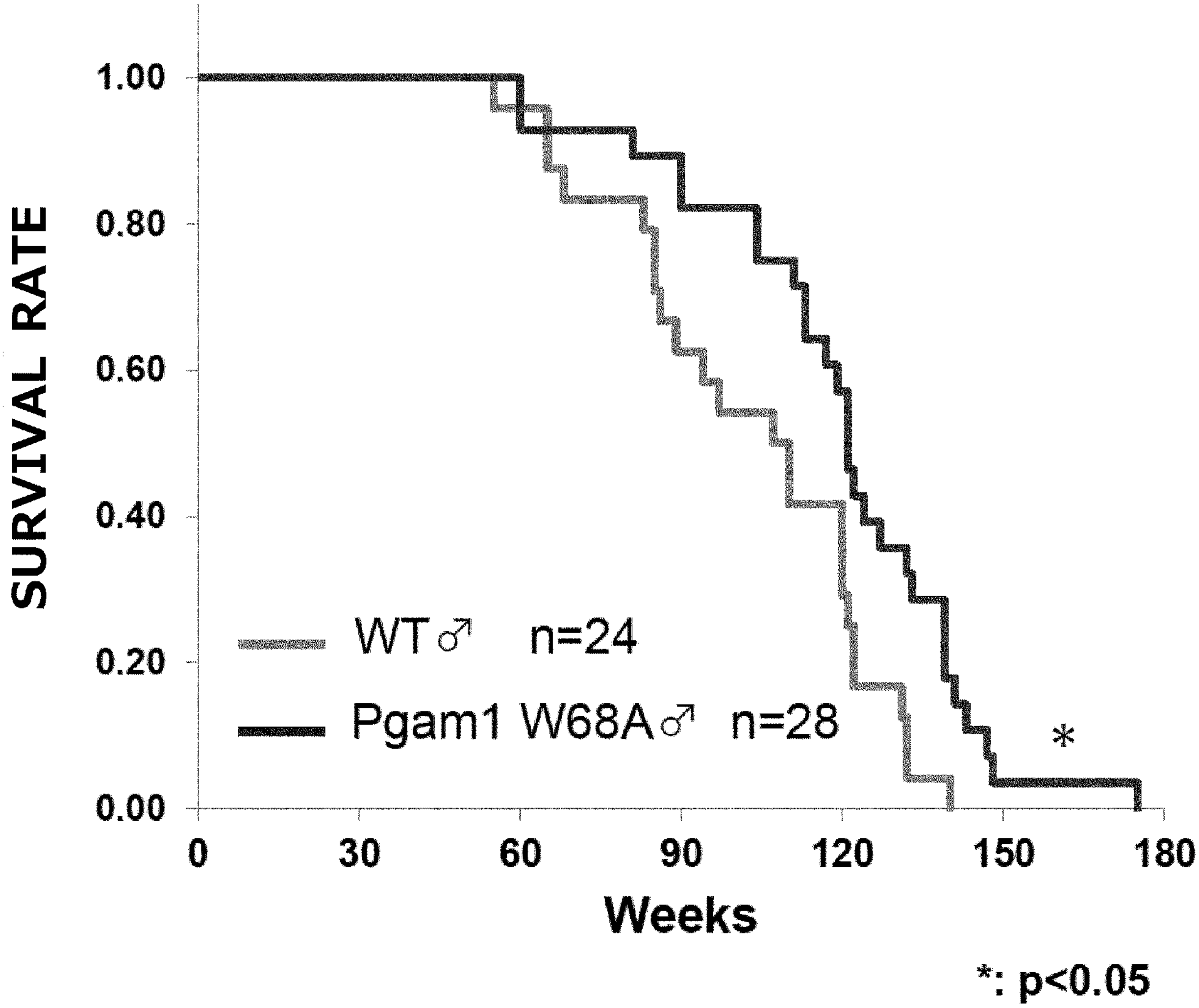


(52) Cooperative Patent Classification (CPC): (Cont.)
**A61K 31/4745; A61K 31/495; A61K 31/496; A61K 31/498; A61K 31/4985; A61K 31/505; A61K 31/506; A61K 31/517; A61K 31/519; A61K 31/5377; A61K 31/635; A61K 31/704; A61K 31/7048; A61K 31/7088; A61K 31/713; A61K 45/00; A61K 48/00; A61P 1/16; A61P 11/00; A61P 43/00**

## Description

### TECHNICAL FIELD

The present invention relates to a therapeutic agent for a lung disease, a hepatic disease, or a renal disease, which contains a PGAM-Chk1 binding inhibitor.

### BACKGROUND ART

In today's society, where the population aging is increasing, the development of a pharmaceutical based on an innovative approach to a wide variety of age-related diseases, in particular, those affecting the lungs, the liver, the kidneys, and the like important to vital functions, is becoming an urgent issue, along with efforts to promote health of the elderly people in general.

For example, pulmonary fibrosis is a disease characterized by diffuse fibroproliferation in the alveolar walls, with dry cough and dyspnea on exertion as the main symptoms. Pulmonary fibrosis, in the narrow sense, refers to idiopathic pulmonary fibrosis, which is a terminal condition of interstitial pneumonia and, and in the broad sense, refers to a coexisting state of pulmonary fibrogenesis and interstitial pneumonia. All interstitial pneumonia can be causes of inducing pulmonary fibrosis.

Interstitial pneumonia is a general term for diseases that cause inflammation mainly in the pulmonary interstitium, and includes those due to specific causes such as infection, collagenosis, radiation, medical agents, and dust, as well as idiopathic interstitial pneumonia due to unknown causes. As idiopathic interstitial pneumonia, diseases such as idiopathic pulmonary fibrosis, nonspecific interstitial pneumonia, idiopathic organizing pneumonia, respiratory bronchiolitis-associated interstitial lung disease, desquamative interstitial pneumonia, acute interstitial pneumonia, and lymphocytic interstitial pneumonia are known, and idiopathic pulmonary fibrosis occurs most frequently and is also simply referred to as pulmonary fibrosis.

In idiopathic pulmonary fibrosis, fibrous connective tissue is diffusely and excessively formed in the pulmonary interstitium, lung function is impaired, and a mean survival time after diagnosis has been reported to be 2.5 to 5 years (Non Patent Literature 1). Since 3 to 15% of patients with idiopathic pulmonary fibrosis have been revealed to have telomere abnormalities, idiopathic pulmonary fibrosis is also considered one of the representative age-related diseases (Non Patent Literature 2). Interstitial pneumonia induced by a specific cause is often cured by removing the cause or administering an antiinflammatory agent such as a steroid. On the other hand, for the treatment of pulmonary fibrosis and interstitial pneumonia accompanied by fibrogenesis, there is currently no effective treatment that improves prognosis although steroids and/or immunosuppressants are generally used. Therefore, there is a strong demand for development of a novel therapeutic drug.

On the other hand, glycolytic metabolism is a general term for the metabolic process of synthesizing a high-energy molecule known as ATP by degrading glucose taken up by cells. Glycolytic metabolism plays an important role in energy metabolism in most of the normal cells and is essential for the survival of individual cells. Because of this, an abnormality in glycolytic metabolism is closely related to human diseases. A decrease in glycolytic metabolism causes neurodegenerative diseases, cardiac dysfunction, diabetes, muscle atrophy, anemia, or the like, whereas some pathological conditions in which glycolytic metabolism is enhanced are known. Examples of the latter conditions include cancer, ischemia (decreased blood flow in tissues), and local inflammation.

The glycolytic metabolic pathway consists of sequential reactions of 10 glycolytic enzymes. The glycolytic enzyme phosphoglycerate mutase PGAM, as the eighth enzyme of the 10 glycolytic enzymes, catalyzes the conversion reaction of 3-phosphoglycerate (3PG) to 2-phosphoglycerate (2PG). The present inventors have found that PGAM binds to Chk1, which is a serine-threonine protein kinase, in cells, that this physical binding plays an important role in glycolytic metabolism, and that the glycolysis can be controlled by modulating this physical binding (Patent Literature 1). However, it is totally unknown about the relationship between the control of glycolytic metabolism and diseases such as fibrosis.

### CITATION LIST

#### Patent Literature

Patent Literature 1: JP 2018-194299 A

#### Non Patent Literature

Non Patent Literature 1: Pharmacol Ther. 2015 May 3. pii: S0163-7258(15)00091-1
Non Patent Literature 2: Mutation Research, 2012, 730(1-2), 52-58

SUMMARY OF INVENTION

Technical Problem

Under the circumstances as described above, an object of the present invention is to provide a novel therapeutic drug that exhibits an excellent therapeutic effect on lung disease, a hepatic disease, or a renal disease, in particular, pulmonary fibrosis and nephrosclerosis.

Solution to Problem

The present inventors have carried out intensive research in order to solve the above problems, and as a result, found that an inhibitor of the physical binding between PGAM, which is a glycolytic metabolic enzyme, and Chk1, which is a serine-threonine protein kinase, exhibits a therapeutic effect on a lung disease, a hepatic disease, and a renal disease. In particular, it has an excellent therapeutic effect on pulmonary fibrosis, which is one of lung diseases. That is, the present invention is as shown below.

[1] A therapeutic agent for a lung disease, a hepatic disease, or a renal disease, containing a PGAM-Chk1 binding inhibitor as an active ingredient.
[2] A therapeutic agent for a lung disease or a renal disease, containing a PGAM-Chk1 binding inhibitor as an active ingredient.
[3] The therapeutic agent according to [2], wherein the lung disease is pulmonary fibrosis.
[4] The therapeutic agent for a lung disease, a hepatic disease, or a renal disease according to [1], wherein the therapeutic agent is a therapeutic agent for a lung disease, a hepatic disease, or a renal disease in elderly people.
[5] The therapeutic agent according to [1] or [2], wherein the binding inhibitor is at least one selected from the group consisting of an RSK1 kinase inhibitor, an Fak kinase inhibitor, an inhibitor of a signal pathway involving Fak kinase, a CDK inhibitor, a calcium antagonist, a cardiac glycoside, a DNA-damaging agent, an antimicrobial agent, an Aurora kinase inhibitor, a flavonoid, a PI3K kinase inhibitor, an HDAC inhibitor, a therapeutic agent for age-related macular degeneration, a p38 MAPK inhibitor, an mTOR inhibitor, a tyrosine kinase inhibitor, a Bcl-2 inhibitor, an HIF-2$\alpha$ inhibitor, a statin, a serotonin receptor antagonist, a further kinase inhibitor, and nutlin 3b.
[6] The therapeutic agent according to [5], wherein

the RSK1 kinase inhibitor is an RSK1 antisense nucleic acid, an RSK1 siRNA, or BI-D1870;
the Fak kinase inhibitor is PF-00562271 or PF-431396;
the inhibitor of a signal pathway involving Fak kinase is PHA-665752 or CP-100356;
the CDK inhibitor is SNS-032, AZD5438, Flavopiridol (Alvocidib), PHA-793887, AT7519, or PHA-767491;
the calcium antagonist is Manidipine or Lomerizine 2HCl;
the cardiac glycoside is Proscillaridin A or Digoxin;
the DNA-damaging agent is Doxorubicin (Adriamycin) HCl, Daunorubicin HCl, Epirubicin HCl, Mitoxantrone 2HCl, or Hydroxy Camptothecine;
the antimicrobial agent is Aminoacridine, Triclosan, or Ethacridine lactate;
the Aurora kinase inhibitor is AMG-900 or JNJ-7706621;
the flavonoid is Chrysin;
the PI3K kinase inhibitor is PF-05212384 or XL147;
the HDAC inhibitor is Trichostatin A (TSA);
the therapeutic agent for age-related macular degeneration is Verteporfin;
the p38 MAPK inhibitor is SB203580 or Doramapimod (BIRB796);
the mTOR inhibitor is AZD8055 or KU-0063794;
the tyrosine kinase inhibitor is Neratinib, Vargatef, or NVP-BHG712;
the Bcl-2 inhibitor is TW-37;
the HIF-2$\alpha$ inhibitor is HIF-2$\alpha$ siRNA;
the statin is Mevastatin;
the serotonin receptor antagonist is RS-127445; and
the further kinase inhibitor is BMS-345541 or CX-4945.

[7] A PGAM-Chk1 binding inhibited mutant knock-in mouse, wherein a mutant Pgam1 gene that encodes a mouse mutant PGAM protein has been knocked-in, and the mouse mutant PGAM protein has a mutation that prevents a PGAM protein, which is a Chk1 binding protein, from binding to a Chk1 protein.

[8] The PGAM-Chk1 binding inhibited mutant knock-in mouse according to [7], wherein a mutant Pgam1 gene that encodes a mouse mutant PGAM protein including the amino acid sequence represented by SEQ ID NO: 11, has been knocked-in.

[9] A life span-extending agent, containing a PGAM-Chk1 binding inhibitor as an active ingredient.

Advantageous Effects of Invention

The therapeutic agent of the present invention exhibits an excellent therapeutic effect on a lung disease, a hepatic disease, or a renal disease, in particular, a lung disease, and especially pulmonary fibrosis, by containing a PGAM-Chk1 binding inhibitor as an active ingredient. The therapeutic agent of the present invention is also expected to have an excellent therapeutic effect on idiopathic pulmonary fibrosis (IPF) and the like for which no effective therapeutic agent has existed until now.

BRIEF DESCRIPTION OF DRAWINGS

[Fig. 1] Fig. 1 is a diagram showing a comparison of PGAM-Chk1 binding inhibited mutant knock-in mice (W68A) with wild-type mice (senescence marker expression in the lung).

[Fig. 2] Fig. 2 is diagrams showing a comparison of the PGAM-Chk1 binding inhibited mutant knock-in mice (W68A) with the wild-type mice (inflammation marker expression in the lung).

[Fig. 3] Fig. 3 is diagrams showing a comparison of the PGAM-Chk1 binding inhibited mutant knock-in mice (W68A) with the wild-type mice (HE staining images of the lung).

[Fig. 4] Fig. 4 is diagrams showing a comparison of the PGAM-Chk1 binding inhibited mutant knock-in mice (W68A) with the wild-type mice (senescence marker and inflammation marker expression in the liver).

[Fig. 5] Fig. 5 is diagrams showing a comparison of the PGAM-Chk1 binding inhibited mutant knock-in mice (W68A) with the wild-type mice (senescence marker and inflammation marker expression in the kidney).

[Fig. 6] Fig. 6 is diagrams showing a comparison of the PGAM-Chk1 binding inhibited mutant knock-in mice (W68A) with the wild-type mice (renal function marker).

[Fig. 7] Fig. 7 is a diagram showing a comparison of the PGAM-Chk1 binding inhibited mutant knock-in mice (W68A) with the wild-type mice (body weight change after administration of bleomycin).

[Fig. 8] Fig. 8 is a diagram showing a comparison of the PGAM-Chk1 binding inhibited mutant knock-in mice (W68A) with the wild-type mice (lung weight change after administration of bleomycin).

[Fig. 9] Fig. 9 is diagrams showing a comparison of the PGAM-Chk1 binding inhibited mutant knock-in mice (W68A) with the wild-type mice (expression of fibrosis-related genes: Col1a2, Col5a2, Fibronetin, and Toropelastin after administration of bleomycin).

[Fig. 10] Fig. 10 is a diagram showing a comparison of the PGAM-Chk1 binding inhibited mutant knock-in mice (W68A) with the wild-type mice (Masson's trichrome staining of the lung tissue after administration of bleomycin).

[Fig. 11] Fig. 11 is a diagram schematically showing the screening method of the present invention.

[Fig. 12] Fig. 12 is a diagram showing results of the primary screening of the present invention.

[Fig. 13-1] Fig. 13-1 is a diagram showing results of the secondary screening of the present invention.

[Fig. 13-2] Fig. 13-2 is a diagram showing results of the secondary screening of the present invention.

[Fig. 14] Fig. 14 is a diagram showing drug candidates for disease therapeutic agents according to the screening method of the present invention.

[Fig. 15] Fig. 15 is a diagram showing effects of nutlin 3b administration to fibrosis model mice (survival rate).

[Fig. 16-1] Fig. 16-1 is a diagram showing effects of nutlin 3b administration to fibrosis model mice (expression of fibrosis-related gene Col3a1 in the lung tissue).

[Fig. 16-2] Fig. 16-2 is a diagram showing effects of nutlin 3b administration to fibrosis model mice (expression of fibrosis-related gene Col5a2 in the lung tissue).

[Fig. 16-3] Fig. 16-3 is a diagram showing effects of nutlin 3b administration to fibrosis model mice (expression of fibrosis-related gene Fibronectin in the lung tissue).

[Fig. 16-4] Fig. 16-4 is a diagram showing effects of nutlin 3b administration to fibrosis model mice (expression of fibrosis-related gene Tropoelastin in the lung tissue).

[Fig. 17-1] Fig. 17-1 is diagrams showing effects of nutlin 3b administration to fibrosis model mice (Masson's trichrome staining in the lung tissue).

[Fig. 17-2] Fig. 17-2 is a diagram showing effects of nutlin 3b administration to fibrosis model mice (quantification of fibrotic regions in Masson's trichrome staining in the lung tissue).
[Fig. 18] Fig. 18 is diagrams showing effects of nutlin 3b administration to fibrosis model mice (expression of FoxM1 and its downstream target genes Plk1, Ccnb2, Aspm, and Cep55 in the lung tissue).
[Fig. 19-1] Fig. 19-1 is a diagram showing effects of nutlin 3b administration to aged mice (HE staining and p21 antibody staining of the lung tissue).
[Fig. 19-2] Fig. 19-2 is a diagram showing an effect of nutlin 3b administration to aged mice (reduction in p21 antibody-positive cells in the lung tissue).
[Fig. 20-1] Fig. 20-1 is a diagram showing an effect of nutlin 3b administration to aged mice (HE staining of the renal tissue and glomerular hyalinization score).
[Fig. 20-2] Fig. 20-2 is a diagram showing an effect of nutlin 3b administration to aged mice (HE staining of the renal tissue and interstitial hyalinization positive rate).
[Fig. 20-3] Fig. 20-3 is diagrams showing effects of nutlin 3b administration to aged mice (senescence marker and SASP marker expression in the renal tissue).
[Fig. 21] Fig. 21 is a diagram showing a comparison of the life span between the PGAM-Chk1 binding inhibited mutant knock-in mice (W68A) and the wild-type mice.

## DESCRIPTION OF EMBODIMENTS

Hereinafter, the present invention will be described in detail.

### <Therapeutic agent for lung disease, hepatic disease, or renal disease>

The therapeutic agent of the present invention is characterized by containing a PGAM-Chk1 binding inhibitor as an active ingredient.

In the present invention, PGAM is an enzyme, referred to as phosphoglycerate mutase, that is responsible for the eighth reaction among the 10 sequential reactions of glycolytic enzymes in the glycolytic metabolic pathway and that catalyzes the conversion reaction of 3-phosphoglycerate (3PG) to 2-phosphoglycerate (2PG). PGAM is responsible for a connection between cellular senescence and canceration. PGAM has two isoforms, PGAM1 and PGAM2. PGAM1 and PGAM2 have different tissue expression patterns, but have high homology in terms of amino acid sequence, and are considered to be equivalent in function and enzyme activity. The amino acid sequences of human PGAM1 and PGAM2 are shown as SEQ ID NOs: 1 and 2, respectively, and the cDNA sequences are shown as SEQ ID NOs: 3 and 4, respectively, in the sequence listing.

In the present invention, Chk1 is a serine-threonine protein kinase, and is a checkpoint kinase that plays a role in activating a DNA damage checkpoint in the G2 phase of the cell cycle. Chk1 was found by the present inventors to also play an important role in glycolytic metabolism. The amino acid sequence of human Chk1 is shown as SEQ ID NO: 5, and the cDNA sequence is shown as SEQ ID NO: 6, in the sequence listing.

Glycolytic metabolism in the present invention is a general term for the metabolic process of synthesizing ATP by degrading glucose taken up by cells. There are two pathways in glycolytic metabolism: a pathway that degrades glucose to pyruvate as an intermediate thereof to synthesize lactate, and a pathway that leads to mitochondrial oxidative phosphorylation. The former is called anaerobic glycolytic metabolism because it does not require oxygen respiration, and the latter is called aerobic glycolytic metabolism because it synthesizes ATP by mitochondrial oxygen respiration. This glycolytic metabolism plays an important role in energy metabolism in most of the normal cells and is essential for the survival of an individual.

PGAM and Chk1 in the present invention also include a protein functionally equivalent to the known PGAM and Chk1 described above. Examples of such a protein include, but are not limited to, mutants, alleles, variants, and homologues of PGAM and Chk1, partial peptides of PGAM and Chk1, a fusion protein with a further protein, and one labeled with a tag.

Examples of the mutants of PGAM and Chk1 in the present invention include a naturally occurring protein that consists of an amino acid sequence derived from the amino acid sequence described above by substituting, deleting, inserting, and/or adding one or more amino acids and that is functionally equivalent to a protein consisting of the amino acid sequence described above. In addition, examples of the mutant of each of PGAM and Chk1 also include a protein that is encoded by a naturally occurring DNA that hybridizes with a DNA consisting of the base sequence described above under a stringent condition and that is functionally equivalent to the protein consisting of the amino acid sequence described above.

In the present invention, the number of amino acids to be mutated is not particularly limited, and is usually considered to be 30 amino acids or less, preferably 15 amino acids or less, more preferably 5 amino acids or less (for example, 3 amino acids or less). The amino acid residue to be mutated is desirably mutated to another amino acid that conserves the properties of an amino acid side chain. Examples of the properties of an amino acid side chain include a hydrophobic amino acid (A, I, L, M, F, P, W, Y, or V), a hydrophilic amino acid (R, D, N, C, E, Q, G, H, K, S, or T), an amino acid having an aliphatic side chain (G, A, V, L, I, or P), an amino acid having a hydroxyl group-containing side chain (S, T, or Y), an amino acid having a sulfur atom-containing side chain (C or M), an amino acid having a carboxylic acid- and amide-containing side chain (D, N, E, or Q), an amino acid having a base-containing side chain (R, K, or H), and an amino acid having an aromatic-containing side chain (H, F, Y, W) (each letter in parentheses represents a one-letter code of an amino acid). It is already known that a polypeptide having an amino acid sequence derived from a certain amino acid sequence by modification by deletion, addition, and/or substitution of one or more amino acid residues with another amino acid sequence maintains the biological activity thereof.

"Functionally equivalent" in the present invention means that the target protein has a biological function or a biochemical function equivalent to that of PGAM or Chk1. The biological function or biochemical function of PGAM is a function as a glycolytic enzyme, and specific examples thereof include the function of catalyzing the conversion reaction of 3-phosphoglycerate (3PG) to 2-phosphoglycerate (2PG). In addition, examples of the biological function or biochemical function of Chk1 include a function as a serine-threonine protein kinase.

Examples of a method well known to those skilled in the art for preparing a DNA encoding a protein functionally equivalent to the target protein include a method that uses a hybridization technique or a polymerase chain reaction (PCR) technique. That is, it can be common practice for those skilled in the art to isolate a DNA having high homology to PGAM or Chk1 by using the base sequence of PGAM or Chk1 or a portion thereof as a probe and using an oligonucleotide that specifically hybridizes to PGAM or Chk1 as a primer. As described above, a DNA encoding a protein having a function equivalent to that of PGAM or Chk1, which can be isolated by a hybridization technique or a PCR technique, is also included in the DNA of the present invention.

The state of PGAM and Chk1 in the present invention is not particularly limited, and may be, for example, a purified state, an intracellularly expressed state, or a state expressed in a cell extract.

The biological species from which PGAM and Chk1 in the present invention are derived are not particularly limited, and examples thereof include a human, a monkey, a dog, a cat, a mouse, a rat, a guinea pig, a pig, a cow, a yeast, and an insect, among which, a human, a dog, and a cat, which can be a target of the therapeutic agent, are preferable, and a human is more preferable.

The PGAM-Chk1 binding inhibitor contained in the therapeutic agent of the present invention as an active ingredient may be any compound as long as the compound inhibits the binding between PGAM and Chk1, and may be any of single substances such as a natural compound, an organic compound, an inorganic compound, a nucleic acid, a protein (including an antibody), or a peptide; an expression product of a compound library, a nucleic acid library, a peptide library, or a gene library; and an extract such as a cell extract, cell culture supernatant, a fermented microbial product, a marine organism extract, a plant extract, a prokaryotic cell extract, an eukaryotic single cell extract, or an animal cell extract, and the like.

In the present invention, a method for determining whether a specific substance inhibits the binding between PGAM and Chk1 is not particularly limited, and the determination can be carried out according to the state of PGAM and Chk1. For example, if PGAM and Chk1 are in a purified state, determination can be carried out by adding a test substance to the purified preparation. In addition, if PGAM and Chk1 are in an intracellularly expressed state or in a state expressed in a cell extract, determination can be carried out by adding a test substance to a culture medium of cells or an extract of the cells, or by direct administration thereof to an experimental animal. When the test substance is a protein, determination can also be carried out, for example, by introducing a vector including a DNA encoding the protein into cells expressing PGAM and Chk1, or adding the vector to an extract of cells expressing PGAM and Chk1. In addition, determination can be carried out by using a two-hybrid method that uses, for example, a yeast or animal cells.

In the present invention, when determining whether a specific substance inhibits the binding between PGAM and Chk1, PGAM and/or Chk1 may be contacted with a test substance, by adding the test substance to a sample including PGAM and Chk1. In addition, by adding a test substance to a sample including either PGAM or Chk1 and then adding the other that has not been included, each of these may be contacted with the test substance.

In the present invention, when determining whether a specific substance inhibits the binding between PGAM and Chk1, the binding level between PGAM and Chk1 is measured after the above contacting. The binding level between PGAM and Chk1 when the test substance is not added to the sample including PGAM and Chk1 is measured and used it as a control. Specifically, the binding levels between PGAM and Chk1 when a test substance is added (when contacted) and when the test substance is not added (when not contacted) are measured, and the values thereof are compared. If the binding level between PGAM and Chk1 decreases when a test substance is added as compared with when the test substance is not added, it can be determined that the test substance has the effect of inhibiting the binding between PGAM and Chk1 (has inhibitory activity on the binding between PGAM and Chk1).

Specific examples of a method for measuring the binding level between PGAM and Chk1 include the following method. PGAM2-FLAG protein is immunoprecipitated from a protein extract of cells co-expressing PGAM2-FLAG and Chk1-myc-His expression vectors, by a FLAG tag antibody conjugated to protein G agarose. At this time, Chk1-myc-His protein coprecipitated by binding to the PGAM2-FLAG protein is detected by Western blotting and quantified. Alternatively, the Chk1-myc-His protein is precipitated from the same protein extract, by a Ni-NTA agarose bead. At this time, PGAM2-FLAG protein coprecipitated by binding to the Chk1-myc-His protein is detected by Western blotting and quantified.

Examples of a preferred method for measuring the binding level between PGAM and Chk1 include, in addition to the above, a method that uses PGAM and Chk1 labeled with tags that can bind to each other, and specific examples thereof include the following method that uses a Nanobit system. In Nanobit, a large subunit and a small subunit of a photoprotein prepared based on a luciferase are attached as tags to PGAM and Chk1, respectively. These tags have structures that can bind to each other. Each subunit itself does not emit light, but the binding between PGAM and Chk1 causes the large subunit and the small subunit to associate to form a complete photoprotein, thereby emitting a luminescence signal. By measuring the intensity of the luminescence signal at this time, the binding level can be measured with high sensitivity and high quantitativity.

The PGAM-Chk1 binding inhibitor contained in the therapeutic agent of the present invention as an active ingredient is preferably a compound further having senolytic activity. It has also been reported that senescent cells are involved in age-related diseases, particularly pulmonary fibrosis, among lung diseases, hepatic diseases, and renal diseases, and that the symptoms of pulmonary fibrosis are improved by removing senescent cells. Therefore, a compound having senolytic activity in addition to inhibitory activity on the binding between PGAM and Chk1 is considered to have an excellent therapeutic effect on age-related diseases such as pulmonary fibrosis.

Here, senolysis (removal of senescent cells) is a concept proposed based on the idea that organismal aging can be improved by removing senescent cells, which are a cause of chronic inflammation. The senolytic activity in the present invention refers to the degree of ability to selectively remove senescent cells.

The senolytic activity of a compound can be confirmed by the following method. The toxic activity of a test substance on young cells and toxic activity thereof on senescent cells are measured, and a test substance having no toxic activity on young cells and having toxic activity on senescent cells can be confirmed to have senolytic activity. In addition, when the toxic activity of a test substance on young cells and the toxic activity thereof on senescent cells are measured, and a test substance has remarkably higher toxic activity on senescent cells than the toxic activity on young cells, and then the test substance can be confirmed to have excellent senolytic activity.

Here, in the present invention, examples of the young cells as cultured mouse cells include primary mouse fibroblasts (MEFs; mouse embryonic fibroblasts, or REFs; rat embryonic fibroblasts) and primary mouse skin keratinocytes. In addition, in the present invention, examples of the young cells as cultured human cells include human embryonic lung fibroblasts (IMR90, WI-38, TIG, or MRC cells), primary human skin cells (NHDF cells, BJ cells, or NHEK cells), normal human mammary epithelial cells (NHMEC cells), normal human vascular endothelial cells (HUVEC cells, HAEC cells, HPAEC cells, or HCAEC cells), and normal human prostate epithelial cells (HPEC). Depending on the cell type, the speed of proliferation thereof differs, and the speed of senescence thereof also differs, but in general, it is known that mouse cells become senescent cells in about one month of continuous culture after initial establishment and that human cells become senescent cells in about three months thereof. Therefore, mouse cells within 2 to 3 weeks of continuous culture after initial establishment can be referred to as young cells, and human cells within 1 month of continuous culture after initial establishment can be referred to as young cells. On the other hand, in the present invention, examples of the senescent cells include those cultured beyond the continuous culture period described above, those cultured for 3 to 4 weeks or more for a mouse, and 1 to 2 months or more for a human. Furthermore, even young cells of a mouse or a human can also be caused to transition into senescent cells in a short period of time by a stress that induces cellular senescence (oxidative stress, carcinogenic stress, DNA damage stress, or the like).

The therapeutic agent of the present invention preferably contains a compound having inhibitory activity on the binding between PGAM and Chk1 and senolytic activity as an active ingredient, and preferred examples thereof include an RSK1 kinase inhibitor, an Fak kinase inhibitor, an inhibitor of a signal pathway involving Fak kinase, a CDK inhibitor, a calcium antagonist, a cardiac glycoside, a DNA-damaging agent, an antimicrobial agent, an Aurora kinase inhibitor, a flavonoid, a PI3K kinase inhibitor, an HDAC inhibitor, a therapeutic agent for age-related macular degeneration, a p38 MAPK inhibitor, an mTOR inhibitor, a tyrosine kinase inhibitor, a Bcl-2 inhibitor, an HIF-2$\alpha$ inhibitor, a statin, a serotonin receptor antagonist, a further kinase inhibitor, and nutlin 3b.

(RSK1 kinase inhibitor)

RSK1 is an RSK (ribosomal S6 kinase) family member and a growth factor-regulated serine-threonine kinase. RSK1 has two different kinase catalytic domains and phosphorylates a variety of substrates, including a member of the mitogen-activated kinase (MAPK) signaling pathway. The RSK1 kinase inhibitor contained in the therapeutic agent of the present invention is not particularly limited as long as the inhibitor is a substance that inhibits the above RSK1 kinase activity, and preferred examples thereof include a compound such as an antisense nucleic acid complementary to a transcript of a DNA encoding RSK1, an siRNA, or BI-D1870 (CAS number: 501437-28-1).

The antisense nucleic acid used in the present invention is preferably an antisense nucleic acid that suppresses the expression of the RSK1 gene by inhibiting the process of transcription, splicing, or translation. As an embodiment of an antisense sequence, designing an antisense sequence complementary to an untranslated region in the vicinity of the 5' end of an RSK1 mRNA is considered to be effective in inhibiting translation of the gene. However, a sequence complementary to a coding region or a 3'-untranslated region can also be used. As described above, sequences that can be used in the present invention include not only sequences complementary to a translated region but also those complementary to an untranslated region of the gene can be used. As described above, nucleic acids used in the present invention include not only a nucleic acid including an antisense sequence of a translated region but also a nucleic acid including an untranslated region of the gene. The antisense nucleic acid has preferably 90% or more, most preferably 95% or more complementarity to a transcript of the target gene. The length of an antisense RNA that effectively inhibits the expression of the target gene by using an antisense sequence is not particularly limited.

The RSK1 siRNA used in the present invention means a double-stranded RNA consisting of a short chain within a range that does not exhibit toxicity in cells, and can be, for example, 15 to 49 base pairs, preferably 15 to 35 base pairs, and more preferably 21 to 30 base pairs in length.

The RSK1 siRNA need not be completely identical to the RSK1 gene, and has at least 70% or more, preferably 80% or more, more preferably 90% or more, and most preferably 95% or more sequence homology. A double-stranded RNA portion in which RNAs are paired in the siRNA is not limited to one in which RNAs are perfectly paired, and may include an unpaired portion due to a mismatch (a corresponding base is not complementary), a bulge (one strand has no corresponding base), or the like. In the present invention, both a bulge and a mismatch may be included in a double-stranded RNA region in which RNAs are paired in a dsRNA.

In the present invention, examples of the RSK1 siRNA include one including the sequence of SEQ ID NO: 7 or SEQ ID NO: 8. The siRNA in the present invention may have two overhanging bases such as dTdT added to the 3' end for the purpose of improving the resistance to nuclease degradation or the like.

(Fak kinase inhibitor)

Fak (focal adhesion kinase) is a non-receptor protein tyrosine kinase and is involved in signaling from an integrin-enriched focal adhesion that mediates cell adhesion with the extracellular matrix. An FAK-promoted signal is known to be involved in the survival of anchorage-dependent cells and to be very important for efficient cell migration in response to growth factor receptor or integrin stimulation. The Fak kinase inhibitor contained in the therapeutic agent of the present invention is not particularly limited as long as the inhibitor is a substance that inhibits the above Fak kinase activity, and preferred examples thereof include PF-00562271 (CAS No. 939791-41-0), PF-431396 (CAS No. 717906-29-1), and salts or derivatives thereof having activity equivalent to that thereof.

(Inhibitor of signal pathway involving Fak kinase)

Examples of the inhibitor of the signal pathway involving Fak kinase contained in the therapeutic agent of the present invention include a c-Met inhibitor and an Mdr-1 inhibitor. Preferred examples of the c-Met inhibitor contained in the therapeutic agent of the present invention include PHA-665752 (CAS No. 477575-56-7), and a salt or a derivative thereof having activity equivalent to that of the compound. In addition, preferred examples of the Mdr-1 inhibitor contained in the therapeutic agent of the present invention include CP-100356 (CAS No. 142715-48-8), and a salt or a derivative thereof that is a compound having activity equivalent to that thereof.

(CDK inhibitor)

Cyclin-dependent kinase (CDK) is a protein that is active only when the protein binds to a cyclin in a group of intranuclear kinases that govern the cell cycle. There are a plurality of CDKs that are activated at different time points in the cell cycle. The activity is controlled by phosphorylation and dephosphorylation of tyrosine and threonine residues of the CDK itself. The CDK inhibitor contained in the therapeutic agent of the present invention is a substance that inhibits or suppresses the activity of such a CDK, and preferred examples thereof include SNS-032 (CDK2/7/9 inhibitor; CAS No. 345627-80-7), AZD5438 (CDK1/2/9 inhibitor; CAS No. 602306-29-6), Flavopiridol (Alvocidib) (CDK1/2/4/6/9 inhibitor; CAS No. 146426-40-6), PHA-793887 (CDK7/9 inhibitor; CAS No. 718630-59-2), AT7519 (CDK1/2/4/6/9 Inhibitor; CAS No. 844442-38-2), PHA-767491 (CDK7/9 inhibitor; CAS No. 942425-68-5), and salts or derivatives thereof that are compounds having activity equivalent to that thereof.

(Calcium antagonist)

The calcium antagonist is an agent that binds to a calcium channel on a cell membrane and inhibits a calcium ion influx into cells. Preferred examples of the calcium antagonist contained in the therapeutic agent of the present invention include Manidipine (CAS No. 89226-50-6), Lomerizine 2HCl (CAS No. 101477-54-7), and salts or derivatives thereof that are compounds having activity equivalent to that thereof.

(Cardiac glycoside)

The cardiac glycoside is a general term for a steroid glycoside used for supraventricular tachycardia such as atrial fibrillation or atrial flutter, congestive heart failure accompanied by edema, or arrhythmia, and preferred examples of the cardiac glycoside contained in the therapeutic agent of the present invention include Proscillaridin A (CAS No. 466-06-8), Digoxin (CAS No. 20830-75-5), and salts or derivatives thereof that are compounds having activity equivalent to that thereof.

(DNA-damaging anticancer agent)

A DNA-damaging anticancer drug is a general term for a type of anti-cancer drug that generates DNA- damagein cancer cells to selectively lead to cell death, and preferred examples of the DNA-damaging anticancer drug contained in the therapeutic agent of the present invention include Doxorubicin (Adriamycin) HCl (CAS No. 25316-40-9), Daunorubicin HCl (CAS No. 23541-50-6), Epirubicin HCl (CAS No. 56390-09-1), Mitoxantrone 2HCl (CAS No. 70476-82-3), Hydroxy Camptothecine (CAS No. 64439-81-2), salts or derivatives thereof that are compounds having activity equivalent to that thereof.

(Antimicrobial agent)

Preferred examples of the antimicrobial agent contained in the therapeutic agent of the present invention include Aminoacridine (CAS No. 90-45-9), Triclosan (CAS No. 3380-34-5), Ethacridine lactate (TAZ activation; CAS No. 1837-57-6), and salts or derivatives thereof that are compounds having activity equivalent to that thereof.

(Aurora kinase inhibitor)

Aurora kinase is an enzyme encoded by the AURKA gene in humans and is a member of the serine/threonine kinase family. Aurora kinase contributes to precise progression of mitosis and meiosis and is essential for cell proliferation. It is known that Aurora kinase activity which is regulated by phosphorylation at one or more site peaked at G2/M phase transition of the cell cycle. Preferred examples of the Aurora kinase inhibitor contained in the therapeutic agent of the present invention include AMG-900 (CAS No. 945595-80-2), JNJ-7706621 (CDK1, 2, Aurora A, B inhibition; CAS No. 443797-96-4), and salts or derivatives thereof that are compounds having activity equivalent to that thereof.

(HIF-2α inhibitor)

HIF-2α is a functional homologue of the transcription factor HIF-1α and is evolutionarily conserved. An HIF-family protein is activated when cells or tissues are exposed to a hypoxic condition. In a hypoxic condition, mitochondrial oxygen respiration is limited, thus forcing cells to rely on glycolytic metabolism for energy production. In a hypoxic environment, activated HIF protein enhances a glycolytic metabolism via upregulation of glycolytic enzymes (Iyer et al., Genes and Development, 1998, Vol 12, p149-62). In a normal oxygen environment, HIF protein is an unstable protein that is susceptible to ubiquitination and degraded, whereas in a hypoxic condition, HIF ubiquitination is inhibited, which stabilizes the protein and enhances the activity. The HIF-2α inhibitor contained in the therapeutic agent of the present invention is not particularly limited as long as the inhibitor is a substance that inhibits the HIF-2α function, and preferred examples thereof include an antisense nucleic acid complementary to a transcript of a DNA encoding HIF-2α, and an siRNA (for example, SEQ ID NO: 9 or SEQ ID NO: 10).

(Nutlin-3b)

Nutlin-3b (CAS No. 675576-97-3) is an optical isomer of Nutlin-3a. The therapeutic agent of the present invention preferably contains Nutlin-3b (CAS No. 675576-97-3) as an active ingredient.

In addition to the above, a flavonoid (Chrysin; CAS No. 480-40-0), a PI3K kinase inhibitor (PF-05212384; CAS No. 1197160-78-3, XL147; CAS No. 934526-89-3), an HDAC inhibitor (Trichostatin A (TSA); CAS No. 58880-19-6), a therapeutic agent for age-related macular degeneration (Verteporfin; CAS No. 129497-78-5), a p38 MAPK inhibitor (SB203580; CAS No. 152121-47-6, Doramapimod (BIRB 796); CAS No. 285983-48-4), an mTOR inhibitor (AZD8055; CAS No. 1009298-09-2, KU-0063794; CAS No. 938440-64-3), a tyrosine kinase inhibitor (Neratinib (HER2, EGFR inhibition); CAS No. 698387-09-6, Vargatef; CAS No. 656247-17-5, NVP-BHG712 (c-Raf, c-Src inhibition); CAS No. 940310-85-0), a Bcl-2 inhibitor (TW-37; CAS No. 877877-35-5), a statin (Mevastatin; CAS No. 73573-88-3), a serotonin receptor antagonist (RS-127445; CAS No. 199864-87-4), and a further kinase inhibitor (BMS-345541; CAS No. 445430-58-0, CX-4945; CAS No. 1009820-21-6), and salts or derivatives thereof can be adopted as active ingredients of the therapeutic agent of the present invention.

In addition to the above active ingredients, the therapeutic agent of the present invention may contain, as other optional ingredients, one or more pharmaceutically, pharmacologically or physiologically acceptable carriers, excipients, binders, disintegrants, lubricants, dispersants, surfactants, plasticizers, suspending agents, emulsifiers, diluents, buffering agents, antioxidants, bacterial inhibitors, or the like. In addition, the therapeutic agent may further include a low-molecular-weight polypeptide, a protein such as serum albumin, gelatin, or an immunoglobulin, an amino acid, a sugar such as a polysaccharide and a monosaccharide, a carbohydrate, or a sugar alcohol. The therapeutic agent of the present invention may further contain other active ingredients as necessary.

The therapeutic agent of the present invention can be produced by any conventionally known method in the field of a pharmaceutical, a quasi-drug, a food, or the like. In the production process thereof, the above active ingredients and other optional ingredients may be added and mixed by any known method.

When formed into an aqueous solution for injection is obtained, the therapeutic agent may be used in combination with, for example, an isotonic solution including physiological saline, glucose or other adjuvants such as D-sorbitol, D-mannose, D-mannitol, or sodium chloride, or a suitable dissolution aid such as an alcohol (ethanol or the like), a polyalcohol (propylene glycol, PEG, or the like), or a nonionic surfactant (polysorbate 80, HCO-50). A diluent, a dissolution aid, a pH adjuster, a soothing agent, a sulfur-containing reducing agent, an oxidation inhibitor, or the like may be further contained as necessary.

In addition, the therapeutic agent can also be encapsulated in a microcapsule (microcapsule of hydroxymethylcellulose, gelatin, poly[methylmethacrylic acid], or the like), or formed into a colloidal drug delivery system (liposome, albumin microsphere, microemulsion, nanoparticle, nanocapsule, or the like). Furthermore, a method for forming an agent into a sustained-release agent is also known and can be applied to the present invention.

When the therapeutic agent of the present invention is used as a medicament for humans or other animals, other than direct administration of them to a patient, it is also possible to formulate the therapeutic agent as a formulation by a known pharmaceutical method and administer the formulation. In the case of the formulation thereof, the pharmaceutically acceptable ingredients described above may be added.

Every agent in the present invention can be administered in the form of a pharmaceutical, and can be administered orally or parenterally systemically or locally. For example, intravenous injection such as drip infusion, intramuscular injection, intraperitoneal injection, subcutaneous injection, a suppository, enema, or an oral enteric preparation can be selected, and the administration method can be appropriately selected according to the age and the symptom of the patient. The effective dosage is selected in the range of 0.001 mg to 100 mg per kg body weight per dose. Alternatively, a dosage of 0.1 to 1000 mg, preferably 0.1 to 50 mg per patient can be selected. As a specific example, 0.1 mg to 40 mg, preferably 1 mg to 20 mg per kg of body weight per month (4 weeks) is divided into one to several times, and administered by a method such as intravenous injection such as drip infusion, subcutaneous injection, or oral administration, on an administration schedule of, for example, twice/week, once/week, once/2 weeks, or once/4 weeks. The administration schedule can also be adjusted by, for example, extending the administration interval from twice/week, or once/week to once/2 weeks, once/3 weeks, once/4 weeks, or the like while monitoring the physical conditions and monitoring trends in blood test values after administration.

The therapeutic agent of the present invention exhibits an excellent function improving effect in the lung, the liver, the kidney, or the like, and is preferably used as a therapeutic agent for a lung disease, a hepatic disease, or a renal disease, more preferably used as a therapeutic agent for a lung disease or a renal disease, and, in particular, further preferably used as a therapeutic agent for pulmonary fibrosis and a therapeutic agent for nephrosclerosis. Examples of pulmonary fibrosis include diseases accompanied by pulmonary fibrosis, such as interstitial pneumonia, idiopathic pulmonary fibrosis, pulmonary fibrosis due to chronic or acute rejection after lung transplantation, pulmonary fibrosis post COVID-19 sequelae, nonspecific interstitial pneumonia, idiopathic organizing pneumonia, desquamative interstitial pneumonia, respiratory bronchiolitis-associated interstitial pneumonia, acute interstitial pneumonia, lymphocytic interstitial pneumonia, sarcoidosis, chronic eosinophilic pneumonia, acute eosinophilic pneumonia, lymphangioleiomyomatosis, pulmonary alveolar proteinosis, Hermansky-Pudlak syndrome, pulmonary Langerhans cell histiocytosis, siderosis, amyloidosis, pulmonary alveolar microlithiasis, hypersensitivity pneumonitis, pneumoconiosis, infectious lung disease, drug-induced pneumonia, and radiation pneumonitis. The therapeutic agent of the present invention is particularly preferably used as a therapeutic agent for a lung disease, a hepatic disease, or a renal disease in elderly people. Further, as described later, PGAM-Chk1 binding inhibited mutant knock-in mice have a longer life span than wild-type mice, and thus, the therapeutic agent of the present invention has effects of extending life span, suppressing senescence, and the like, and can also be effective as a life span-extending agent, an anti-senescent agent, a senescence inhibitor, and a senescence retarder.

<PGAM-Chk1 binding inhibited mutant knock-in mice>

The PGAM-Chk1 binding inhibited mutant knock-in mice of the present invention are mice in which PGAM protein cannot bind to CHK1 protein due to the mutation of Pgam1 genes. In the mice, the amino acid constituting the binding site of the PGAM protein to the CHK1 protein is substituted with another amino acid, and thereby, the PGAM protein cannot bind to the CHK1 protein. The above amino acid substitution is not particularly limited as long as it makes the PGAM protein unable to bind to the CHK1 protein, and preferred examples thereof include the one in which the 68th tryptophan of the PGAM protein is substituted with alanine. Specifically, the PGAM-Chk1 binding inhibited mutant knock-in mice of the present invention are mice in which a mutant Pgam1 gene that encodes a mouse mutant PGAM protein consisting of the amino acid sequence represented by SEQ ID NO: 11, has been knocked-in.

Examples of the protein in which the amino acid constituting the binding site of the PGAM protein to the CHK1 protein is substituted with another amino acid, and thereby, the PGAM protein cannot bind to the CHK1 protein, can include a protein that consists of an amino acid sequence derived from the amino acid sequence represented by SEQ ID NO: 11 by substituting, deleting, inserting, and/or adding one or more amino acids and that is functionally equivalent to a protein consisting of the amino acid sequence represented by SEQ ID NO: 11. The amino acid sequences of the proteins have the above 68th tryptophan substituted with alanine and need to have glycolytic enzyme phosphoglycerate mutase, PGAM.

The PGAM-Chk1 binding inhibited mutant knock-in mice of the present invention also include mice in which a gene (mutant Pgam1 gene) has been knocked in, the gene encoding a mouse mutant PGAM protein, which is a protein consisting of an amino acid sequence derived from the amino acid sequence represented by SEQ ID NO: 11 by substituting, deleting, inserting, and/or adding one or more amino acids and is functionally equivalent to a protein consisting of the amino acid sequence represented by SEQ ID NO: 11.

The PGAM-Chk1 binding inhibited mutant knock-in mice of the present invention have no abnormalities in health and are normal in fertility when young; however, the difference from the wild-type mice becomes apparent in their senile stage of life. For example, they can be said to be mice in which the expression of senescence and inflammation markers in each organ (a lung, a liver, a kidney, and the like), which are known to increase with age, symptoms of chronic inflammation, and the like are suppressed, thereby the senescent progression is suppressed. The mice have a longer life span than the wild-type mice, and tend to live longer.

The PGAM-Chk1 binding inhibited mutant knock-in mice of the present invention are preferably used as an experimental system to verify the therapeutic effects of inhibiting PGAM-Chk1 binding on various age-related diseases or pathological conditions in which the PGAM-Chk1 binding is enhanced.

## EXAMPLES

The present invention will be specifically described with reference to the following Examples, but the present invention is not limited by the Examples.

### 1. Preparation of C57BL/6J PGAM-Chk1 binding inhibited mutant knock-in mice

The 68th tryptophan in Pgam1 genes of mouse fertilized eggs was substituted with alanine using the CRISPA-Cas9 system. The fertilized eggs that had been cultured to the 2-cell stage were transplanted to foster mothers (ICR 8-week old), who gave birth later. DNA was extracted from the auricles after birth, and the genotype was determined.

### 2. Characteristics of PGAM-Chk1 binding inhibited mutant knock-in mice

PGAM-Chk1 binding inhibited mutant knock-in mice prepared by the above method had no abnormalities in their health, and their fertility was normal at a young age. Under normal breeding conditions, differences from wild-type mice were only apparent at an older age.

The expression of inflammation and senescence markers in the lung tissue was compared between C57BL/6J, 90-week old wild-type (+/+) and the above knock-in mice (KI/KI). The relative expression levels of p16Ink4 mRNA as a senescence marker, and IL1$\alpha$ and IL6 mRNA as inflammation markers were measured. As shown in Fig. 1 (senescence marker) and Fig. 2 (Inflammation marker), the expression of the senescence marker and the inflammation markers in the lung was reduced in the knock-in mice. In addition, the HE staining images of the lung are shown in Fig. 3. In the wild-type (control) aged mice, infiltration of immune cells was observed from the pleural side and chronic inflammation occurred; whereas in the binding mutant knock-in mouse (KI group), no such signs of chronic inflammation were observed.

The expression of inflammation and senescence markers in the liver was compared between C57BL/6J, 90-week old wild-type (+/+) and the above knock-in mice (KI/KI). The relative expression levels of p16Ink4 mRNA as a senescence marker, and IL6 mRNA as an inflammation marker were measured. As shown in Fig. 4, the expression of the senescence marker and the inflammation marker in the liver was reduced in the knock-in mice.

The expression of inflammation and senescence markers in the kidney and the expression of a renal function marker were compared between 90-week old C57BL/6J wild-type (+/+) and the above knock-in mice (KI/KI). The relative expression levels of p16Ink4 mRNA as a senescence marker, and IL6 mRNA as an inflammation marker were measured. As shown in Fig. 5, the expression of the senescence marker and the inflammation marker in the kidney was reduced in the knock-in mice. In addition, in the knock-in mice, the improvement in renal function marker was observed (Fig. 6).

3. Pulmonary fibrosis model by bleomycin (BLM)

2.5 mg/kg bleomycin was intratracheally administered to 20-week old PGAM-Chk1 binding inhibited mutant knock-in mice (KI/KI) and wild-type mice (WT). The mice were checked for survival every day, weighed 14 days after bleomycin administration (Fig. 7), and then sacrificed. The lung tissue was sampled and weighed (Fig. 8). Then, a portion of the lung tissue was immersed in a sufficient amount of RNAlater (manufactured by Invitrogen), and stored at 4°C overnight. The next day, the lung tissue was removed from the RNAlater, and TotalRNA was collected using TRIzole (manufactured by Invitrogen). Then, mRNA was reverse-transcribed using a ReverTra Ace qPCR RT kit (manufactured by TOYOBO Co., Ltd.) to prepare cDNA. Real-time PCR was performed on the prepared cDNA using primers specific to fibrosis-related genes (Col1a2, Col5a2, Fibronectin, and Toropelastin). The results are shown in Fig. 9. Also, some lung tissue was fixed, treated with 4% paraformaldehyde for 48 hours. The fixed lung tissue was embedded in paraffin, then sliced into 5 $\mu$m of thin slices, and set on microscope slides. Masson's trichrome staining was performed on the thin slices of the lung tissue to evaluate fibrosis (Fig. 10).

As shown in Fig. 7, the wild-type mice (WT) lost body weight by intratracheal administration of bleomycin; however, the PGAM-Chk1 binding inhibited mutant knock-in mice (KI/KI) did not lose body weight. Meanwhile, the lung weight increased in the wild-type mice (WT) by intratracheal administration of bleomycin, suggesting the occurrence of pulmonary fibrosis. On the other hand, the lung weight did not change in the PGAM-Chk1 binding inhibited mutant knock-in mice (KI/KI), suggesting recovery from pulmonary fibrosis caused by the administration of bleomycin (Fig. 8). Further, as shown in Fig. 9, the expression of fibrosis-related genes in the lungs of the wild-type mice (WT) was significantly increased by intratracheal administration of bleomycin; whereas, the degree of increase was small in the PGAM-Chk1 binding inhibited mutant knock-in mice (KI/KI) (Fig. 9). Also from the results of the Masson's trichrome staining of the lung tissue slices, lung fibrosis was recovered in the PGAM-Chk1 binding inhibited mutant knock-in mice (KI/KI) compared to wild-type mice (WT).

4. Search for PGAM-Chk1 binding inhibitory substance

PGAM-Chk1 binding inhibition HTP (high-throughput) screening using a Cell-base NanoBiT system was carried out. Specifically, the screening was carried out by the following method. In NanoBiT, a large subunit and a small subunit of a photoprotein prepared based on a luciferase are attached as tags to PGAM and Chk1, respectively. These tags have structures that can bind to each other. Each subunit itself does not emit light, but the binding between PGAM and Chk1 causes the large subunit and the small subunit to associate to form a complete photoprotein, thereby emitting a luminescence signal. By measuring the intensity of the luminescence signal at this time, the binding level can be measured with high sensitivity and high quantitativity. At this time, the luminescence signal decreases in the presence of a substance that inhibits the binding of PGAM and Chk1, such as nutlin. Therefore, a substance that inhibits the binding of PGAM and Chk1 was searched from the drug library, with the decrease in the luminescence signal as an indicator.

By using this PGAM-Chk1 binding visualization Cell-base NanoBiT system, with the support of the Kyoto University Medical Research Support Center, PGAM-Chk1 binding inhibition HTP screening (primary screening) was carried out on a library of existing drugs (more than 2300) having established safety evaluation, and a little over 200 binding inhibitory drug candidates were acquired (Fig. 11 and Fig. 12).

For these drug candidate, the sensitivity of young IMR90 cells and senescent IMR90 cells thereto was compared, and substances with high cytotoxic activity against senescent IMR90 cells and low cytotoxic activity against young IMR90 cells were selected as substances with high senolytic activity and advanced to the next step (secondary screening). The senescent IMR90 cells were prepared by inducing an oncogenic Ras mutation. The young IMR90 cells and the senescent IMR90 cells were each seeded in a 96-well plate, and two days later, treated with the drug candidates obtained above at two concentrations of 5 $\mu$M and 10 $\mu$M. Surviving cells after 72 hours were fixed with formalin and stained with crystal violet. After that, crystal violet in each well was extracted with 2% Triton X100 and quantified (Fig. 13-1 and Fig. 13-2). Young IMR90 cells and senescent IMR90 cells were each treated with a solvent (DMSO), and the resulting cells were used as a negative control, and the survival rate compared to the negative control was calculated by the following expression. Furthermore, the sensitivity of the young IMR90 cells and the senescent IMR90 cells to drug agents was compared, and compounds having a value of young cell viability/senescent cell viability of 1.14 or more were selected.

y/senescent cell viability of 1.14 or more were selected.

$$\text{Viability (\%)} = (\text{Sample/negative subject}) \times 100$$

$$\text{Senolytic activity} = \text{young cell viability/senescent cell viability}$$

As described above, at present, more than 40 candidate compounds for therapeutic agents were obtained, including nutlin 3b and the RSK kinase inhibitor BI-D1870, and agents acquired in the secondary screening, as summarized in Fig. 14. These compounds acquired in the secondary screening were able to be classified into approximately 7 groups by functional classification. As shown in Fig. 11, by further conducting a test using pulmonary fibrosis model animals as a tertiary screening, agents that exhibit therapeutic effects on pulmonary fibrosis can be selected.

5. Examination of effect of nutlin 3b administration to BLM pulmonary fibrosis model mice - 1

(1) Survival rate

2.5 mg/kg of bleomycin was intratracheally administered to 10- to 12-week old C57BL6 mice. Starting 8 days after bleomycin administration, 20 mg/kg of nutlin 3b or the solvent alone was intraperitoneally administered 4 times a week for 2 weeks. The mice were checked for survival every day, and the number of mice that died within 21 days after bleomycin administration was counted. In this test, 6 mice in the nutlin 3b non-administration group (solvent only) and 7 mice in the nutlin 3b administration group were used. The results are shown in Fig. 15. As shown in Fig. 15, administration of nutlin 3b improved the survival rate of the mice pulmonary fibrosis model mice. In the figure, 3B indicates nutlin 3b. The same applies to the following figures.

(2) Expression of fibrosis-related mRNA

2.5 mg/kg of bleomycin was intratracheally administered to 10- to 12-week old ICR mice. Starting 8 days after bleomycin administration, 14, 20, and 24 mg/kg of nutlin 3b or the solvent alone were intraperitoneally administered 4 times a week for 2 weeks. 21 days after bleomycin administration, mice in the nutlin 3b administration group or solvent administration group were sacrificed to sample their lung tissue. In the test, 3 mice in the nutlin 3b non-administration group (solvent only) and 3 mice in the nutlin 3b administration group were used. The same applies to the following tests. The collected lung tissue was immersed in a sufficient amount of RNAlater and stored at 4°C overnight. The next day, the lung tissue was removed from the RNAlater, and the total RNA was collected using TRIzole. Then, mRNA was reverse-transcribed using a ReverTra Ace qPCR RT kit to prepare cDNA. Real-time PCR was performed on the prepared cDNA using primers specific to fibrosis-related genes (Col1a2, Col5a2, Fibronectin, and Toropelastin). The results are shown in Figs. 16-1 to 4.

As shown in Figs. 16-1 to 16-4, the expression of the fibrosis-related genes, which had increased by bleomycin administration, decreased by nutlin 3b administration.

(3) Pathological comparison

2.5 mg/kg of bleomycin was intratracheally administered to 10- to 12-week old ICR mice. Starting 8 days after bleomycin administration, 14, 20, and 24 mg/kg of nutlin 3b or the solvent alone were intraperitoneally administered 4 times a week for 2 weeks. 21 days after bleomycin administration, mice in the nutlin 3b administration group or solvent administration group were sacrificed to sample their lung tissue. The lung tissue was fixed by treatment with 4% paraformaldehyde for 48 hours. The fixed lung tissue was embedded in paraffin, and then sliced into 5 μm thin slices, and set on microscope slides. Masson's trichrome staining was performed on the thin slices of the lung tissue to evaluate fibrosis. Specifically, in Masson's trichrome staining, nuclei are stained black-brown with iron hematoxylin staining, cytoplasm is stained red with nuclear fuchsin, and collagen fibers are stained blue with aniline blue. Accordingly, to evaluate fibrosis, the stained image was separated into the three primary colors using ImageJ (image analysis software), and the area which stained blue was quantified as the fibrosis regions. The obtained signals of each group were expressed as relative values to the control group (PBS + PEG). The results of Masson's trichrome staining are shown in Fig. 17-1, and the quantification results of the fibrosis regions are shown in Fig. 17-2.

As shown in Figs. 17-1 to 2, the fibrosis areas induced by bleomycin in the lungs in the nutlin 3b administration group were smaller than those of the control group, confirming the therapeutic effect on fibrosis.

6. Examination of effect of nutlin 3b administration to BLM pulmonary fibrosis model mice - 2

The influence of the nutlin 3b administration to FoxM1 target factors in bleomycin-induced pulmonary fibrosis was examined. Specifically, 2.5 mg/kg of bleomycin was intratracheally administered to 10- to 12-week old ICR mice. Starting 8 days after bleomycin administration, 20 mg/kg of nutlin 3b or the solvent alone was intraperitoneally administered 4 times a week for 2 weeks. 21 days after bleomycin administration, mice in the nutlin 3b administration group and the solvent administration group were sacrificed to sample their lung tissue. In the test, 3 mice in the nutlin 3b non-administration group (solvent only) and 3 mice in the nutlin 3b administration group were used. The collected lung tissue was immersed in a sufficient amount of RNAlater and stored at 4°C overnight. The next day, the lung tissue was removed from the RNAlater, and the total RNA was collected using TRIzole. Then, mRNA was reverse-transcribed using a ReverTra Ace qPCR RT kit to prepare cDNA. Real-time PCR was performed on the prepared cDNA using primers specific to FoxM1 and its downstream target genes (Plk1, Ccnb2, Aspm, and Cep55). The results are shown in Figs. 18-1 to 18-2.

As shown in Fig. 18, increased expression of FoxM1 and its downstream target genes was observed in BLM pulmonary fibrosis tissue. When nutlin 3b was administered to BLM-induced pulmonary fibrosis, suppression of the expression of FoxM1 target genes was observed.

7. Examination of effect of nutlin 3b administration on the lungs of aged mice

Nutlin 3b was administered to C57BL/6J, 20-month-old aged mice once a week for 3 months. After 3 months, the mice were sacrificed, and HE staining and mRNA expression analysis of the lung tissue were performed. As a comparison subject, the lung tissue of young mice (C57BL/6J, 10-week old) and aged mice with no administration of nutlin 3b (Vehicle) were used. The results are shown in Figs. 19-1 to 19-2.

As shown in Figs. 19-1 to 19-2, in the aged lung tissue (Vehicle group), the number of p21-positive cells, a senescence marker, increased. On the other hand, p21 cells in aged lung tissue were significantly suppressed by nutlin 3b administration.

8. Examination of effect of nutlin 3b administration on the kidneys of aged mice

Nutlin 3b was administered to C57BL/6J, 20-month-old aged mice once a week for 3 months. After 3 months, the mice were sacrificed, and HE staining and mRNA expression analysis of the renal tissue were performed. As a comparison subject, the renal tissue of young mice (C57BL/6J, 10-week old) and aged mice with no administration of nutlin 3b (Vehicle) were used. The results are shown in Figs. 20-1 to 20-3.

Glomerular hyalinization was evaluated using a four-level score based on HE staining of renal tissue, and the interstitial hyalinization positive rate was evaluated, and thereby, a significant improvement in hyalinization in the glomerular and interstitial regions was observed in the nutlin 3b administration group, as shown in Figs. 20-1 to 20-2. Also, the increase in senescence markers and SASP markers induced in aged renal tissue was suppressed by nutlin 3b administration (Fig. 20-3).

9. Examination of life span-extending effect on PGAM-Chk1 binding inhibited mutant knock-in mice (W68A)

Male PGAM-Chk1 binding inhibited mutant knock-in mice (W68A) and wild-type mice were raised in an SPF environment for a long time. The survival curves of the mice were plotted using the Kaplan-Meier method, and the results are shown in Fig. 21.

As shown in Fig. 21, the PGAM-Chk1 binding inhibited mutant knock-in mice (W68A) tended to live longer compared to the wild-type mice.

INDUSTRIAL APPLICABILITY

The therapeutic agent of the present invention exhibits an excellent therapeutic effect on a lung disease, a hepatic disease, or a renal disease, in particular, a lung disease, and especially pulmonary fibrosis, by containing a PGAM-Chk1 binding inhibitor as an active ingredient. It is also expected to have excellent therapeutic effects on idiopathic pulmonary fibrosis (IPF) and other diseases for which no effective therapeutic agent has existed until now.

## Claims

1. A therapeutic agent for a lung disease, a hepatic disease, or a renal disease, comprising a PGAM-Chk1 binding inhibitor as an active ingredient.

**2.** A therapeutic agent for a lung disease or a renal disease, comprising a PGAM-Chk1 binding inhibitor as an active ingredient.

**3.** The therapeutic agent according to claim 2, wherein the lung disease is pulmonary fibrosis.

**4.** The therapeutic agent for a lung disease, a hepatic disease, or a renal disease according to claim 1, wherein the therapeutic agent is a therapeutic agent for a lung disease, a hepatic disease, or a renal disease in elderly people.

**5.** The therapeutic agent according to claim 1 or 2, wherein the binding inhibitor is at least one selected from the group consisting of an RSK1 kinase inhibitor, an Fak kinase inhibitor, an inhibitor of a signal pathway involving Fak kinase, a CDK inhibitor, a calcium antagonist, a cardiac glycoside, a DNA-damaging agent, an antimicrobial agent, an Aurora kinase inhibitor, a flavonoid, a PI3K kinase inhibitor, an HDAC inhibitor, a therapeutic agent for age-related macular degeneration, a p38 MAPK inhibitor, an mTOR inhibitor, a tyrosine kinase inhibitor, a Bcl-2 inhibitor, an HIF-2$\alpha$ inhibitor, a statin, a serotonin receptor antagonist, a further kinase inhibitor, and nutlin 3b.

**6.** The therapeutic agent according to claim 5, wherein

the RSK1 kinase inhibitor is an RSK1 antisense nucleic acid, an RSK1 siRNA, or BI-D1870;
the Fak kinase inhibitor is PF-00562271 or PF-431396;
the inhibitor of a signal pathway involving Fak kinase is PHA-665752 or CP-100356;
the CDK inhibitor is SNS-032, AZD5438, Flavopiridol (Alvocidib), PHA-793887, AT7519, or PHA-767491;
the calcium antagonist is Manidipine or Lomerizine 2HCl;
the cardiac glycoside is Proscillaridin A or Digoxin;
the DNA-damaging agent is Doxorubicin (Adriamycin) HCl, Daunorubicin HCl, Epirubicin HCl, Mitoxantrone 2HCl, or Hydroxy Camptothecine;
the antimicrobial agent is Aminoacridine, Triclosan, or Ethacridine lactate;
the Aurora kinase inhibitor is AMG-900 or JNJ-7706621;
the flavonoid is Chrysin;
the PI3K kinase inhibitor is PF-05212384 or XL147;
the HDAC inhibitor is Trichostatin A (TSA);
the therapeutic agent for age-related macular degeneration is Verteporfin;
the p38 MAPK inhibitor is SB203580 or Doramapimod (BIRB796);
the mTOR inhibitor is AZD8055 or KU-0063794;
the tyrosine kinase inhibitor is Neratinib, Vargatef, or NVP-BHG712;
the Bcl-2 inhibitor is TW-37;
the HIF-2$\alpha$ inhibitor is HIF-2$\alpha$ siRNA;
the statin is Mevastatin;
the serotonin receptor antagonist is RS-127445; and
the further kinase inhibitor is BMS-345541 or CX-4945.

**7.** A PGAM-Chk1 binding inhibited mutant knock-in mouse, wherein a mutant Pgam1 gene that encodes a mouse mutant PGAM protein has been knocked-in, and the mouse mutant PGAM protein has a mutation that prevents a PGAM protein, which is a Chk1 binding protein, from binding to a Chk1 protein.

**8.** The PGAM-Chk1 binding inhibited mutant knock-in mouse according to claim 7, wherein a mutant Pgam1 gene that encodes a mouse mutant PGAM protein including an amino acid sequence represented by SEQ ID NO: 11, has been knocked-in.

**9.** A life span-extending agent, comprising a PGAM-Chk1 binding inhibitor as an active ingredient.

[Fig. 1]

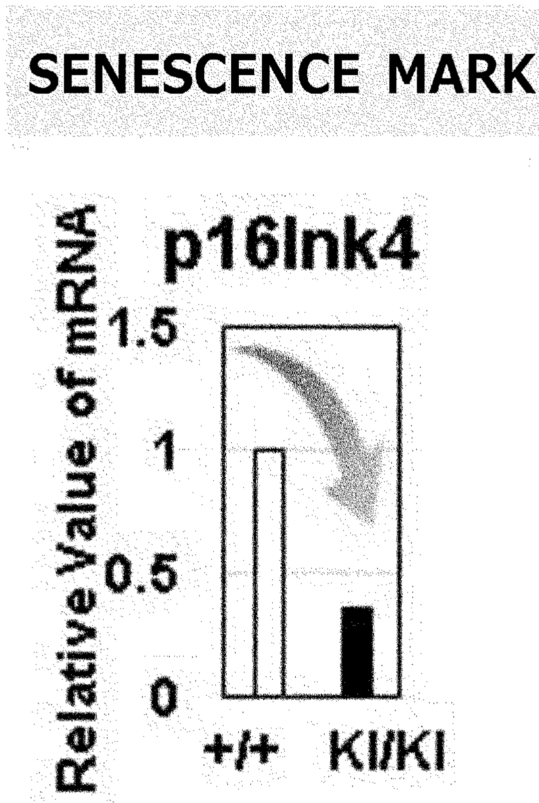
SENESCENCE MARKER
p16Ink4
Relative Value of mRNA
1.5
1
0.5
0
+/+
KI/KI

[Fig. 2]

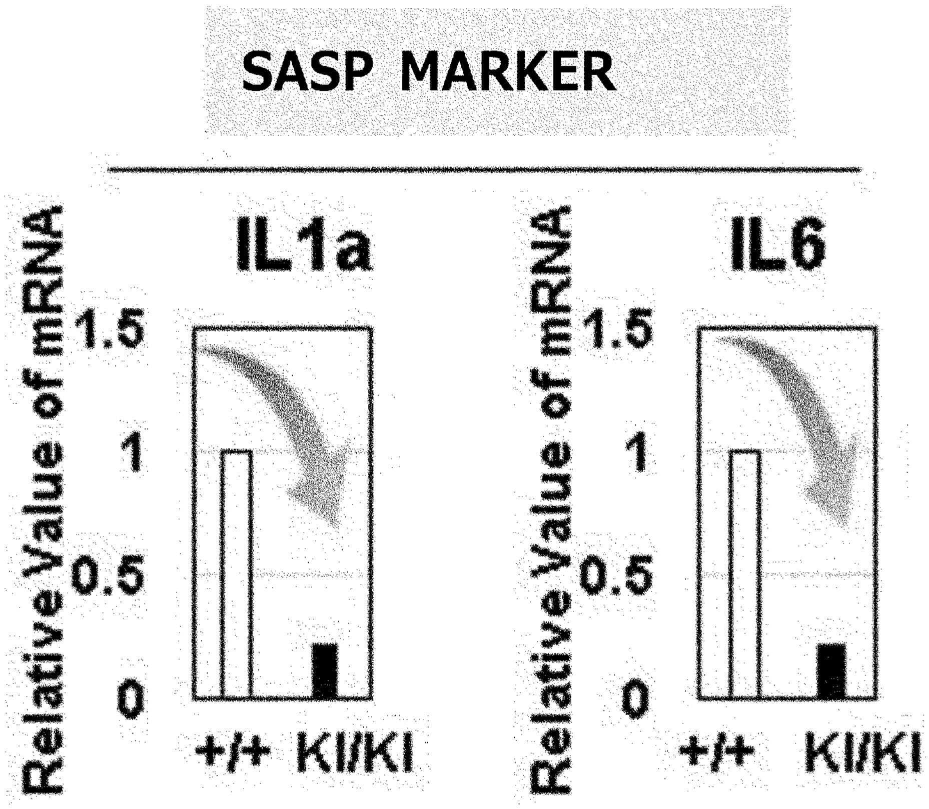
SASP MARKER
IL1a
Relative Value of mRNA
1.5
1
0.5
0
+/+
KI/KI
IL6
Relative Value of mRNA
1.5
1
0.5
0
+/+
KI/KI

[Fig. 3]

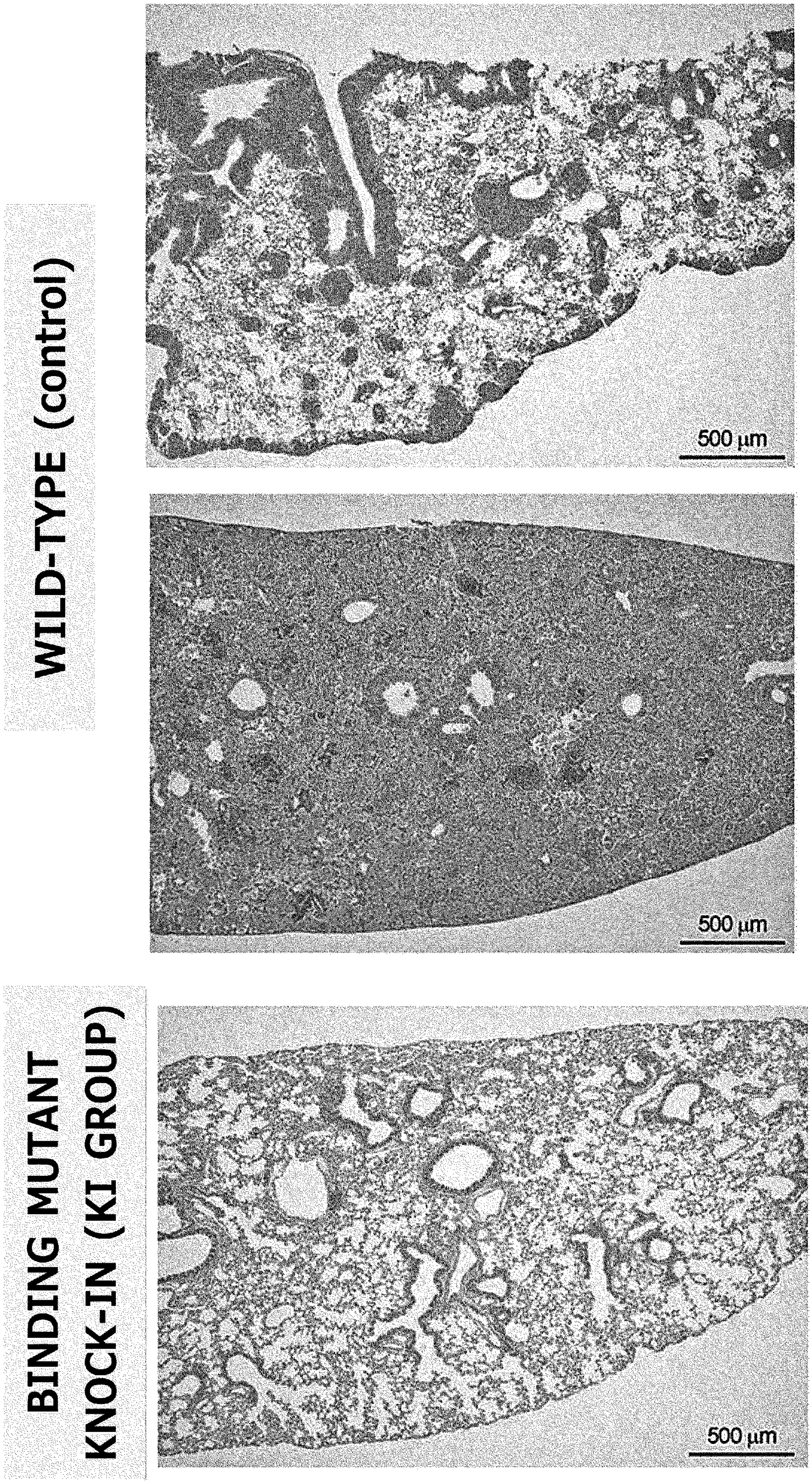
WILD-TYPE (control)
500 µm
500 µm
BINDING MUTANT
KNOCK-IN (KI GROUP)
500 µm

[Fig. 4]

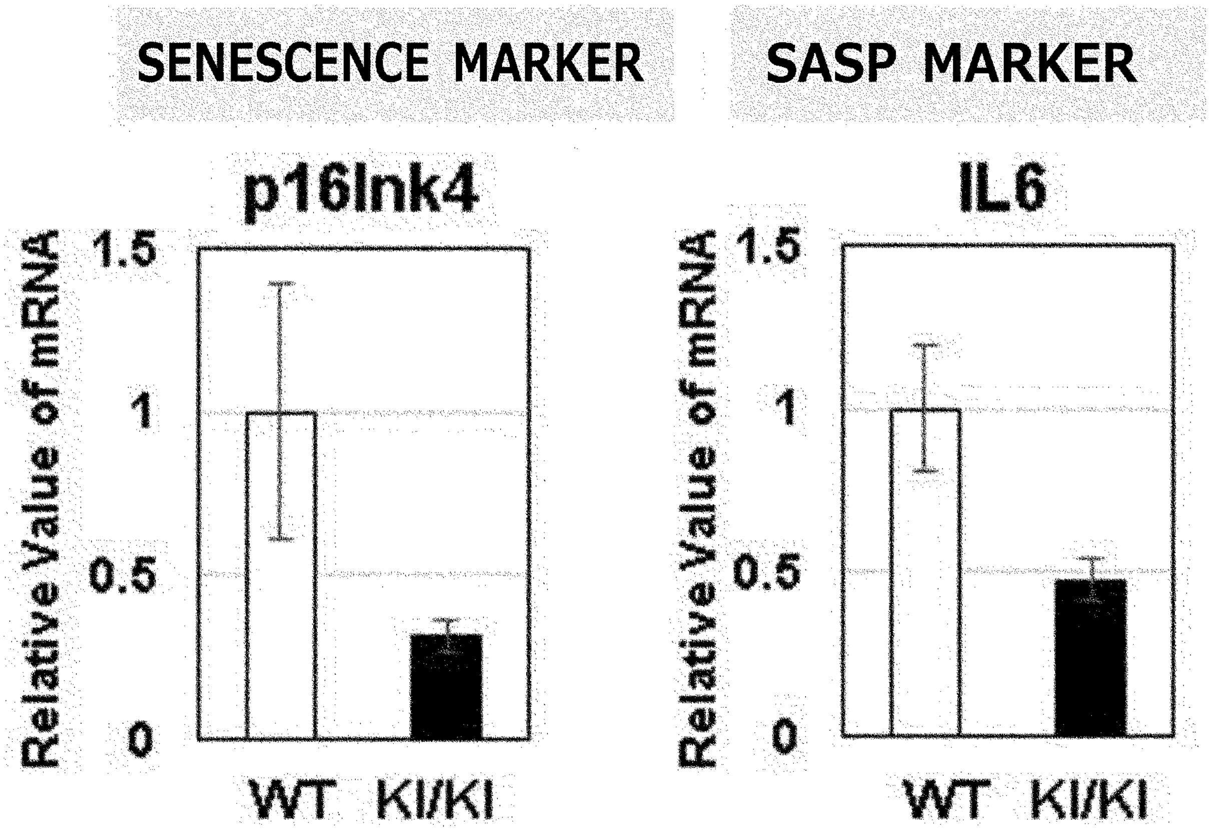
SENESCENCE MARKER
SASP MARKER
p16Ink4
IL6
Relative Value of mRNA
1.5
1
0.5
0
WT
KI/KI

[Fig. 5]

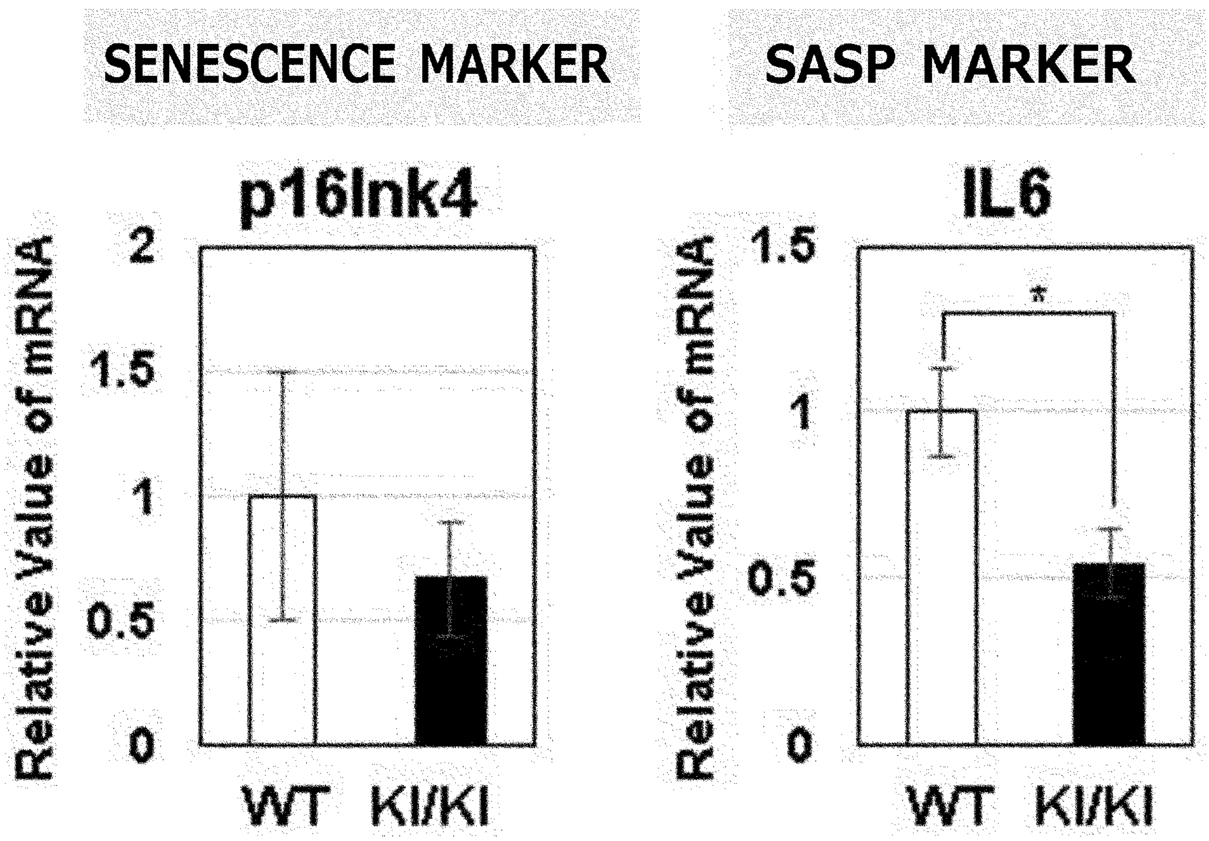
SENESCENCE MARKER
SASP MARKER
p16Ink4
IL6
Relative Value of mRNA
2
1.5
1
0.5
0
*
WT
KI/KI

[Fig. 6]

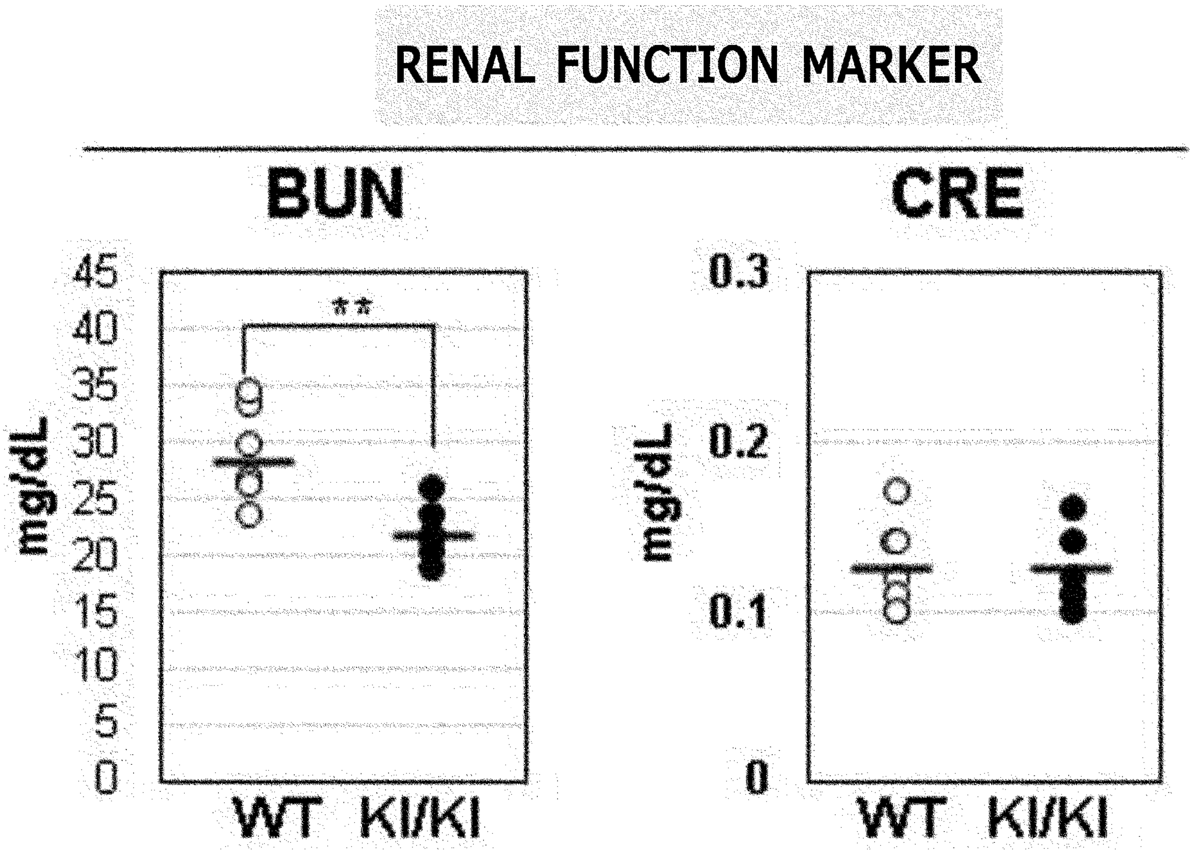
RENAL FUNCTION MARKER
BUN
CRE
mg/dL
45
40
35
30
25
20
15
10
5
0
**
WT
KI/KI
0.3
0.2
0.1
0

[Fig. 7]

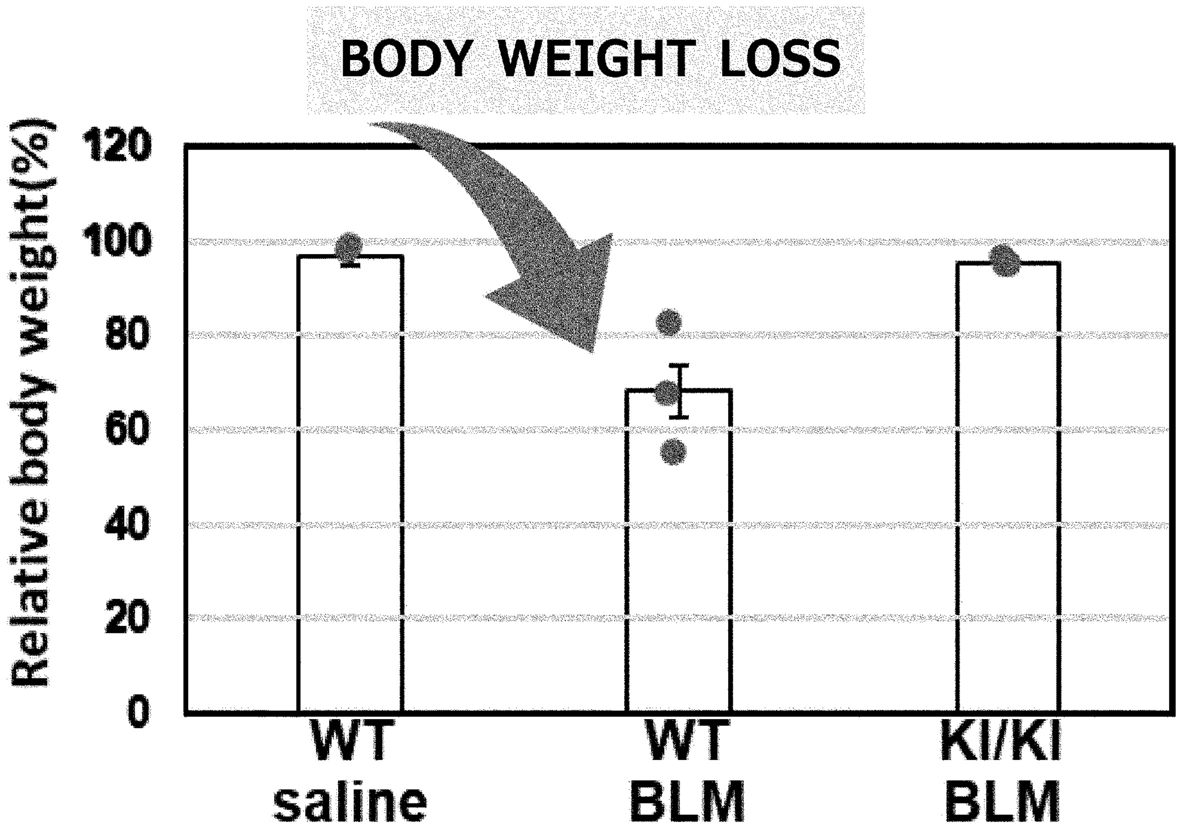
BODY WEIGHT LOSS
Relative body weight(%)
120
100
80
60
40
20
0
WT
saline
WT
BLM
KI/KI
BLM

[Fig. 8]

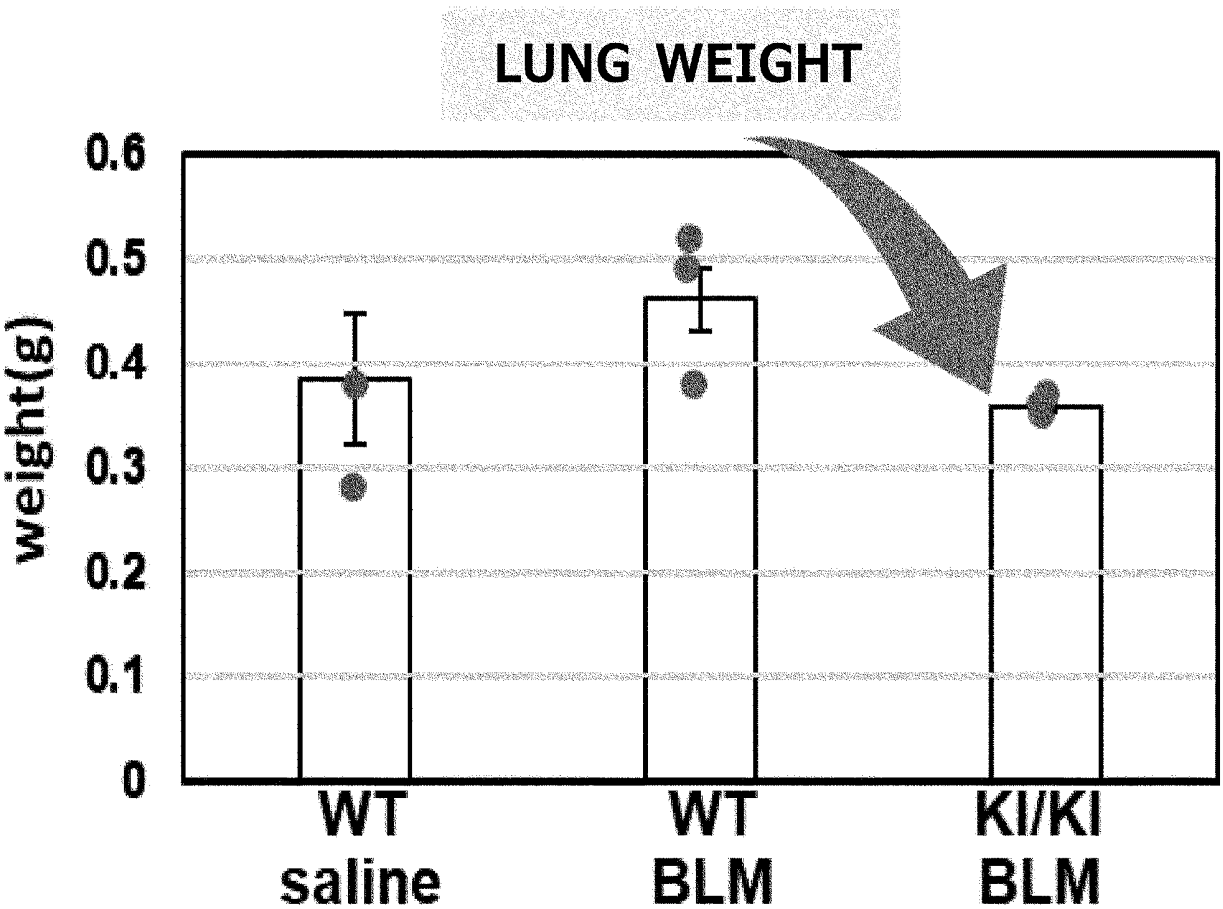
LUNG WEIGHT
0.6
0.5
0.4
0.3
0.2
0.1
0
weight(g)
WT
saline
WT
BLM
KI/KI
BLM

[Fig. 9]

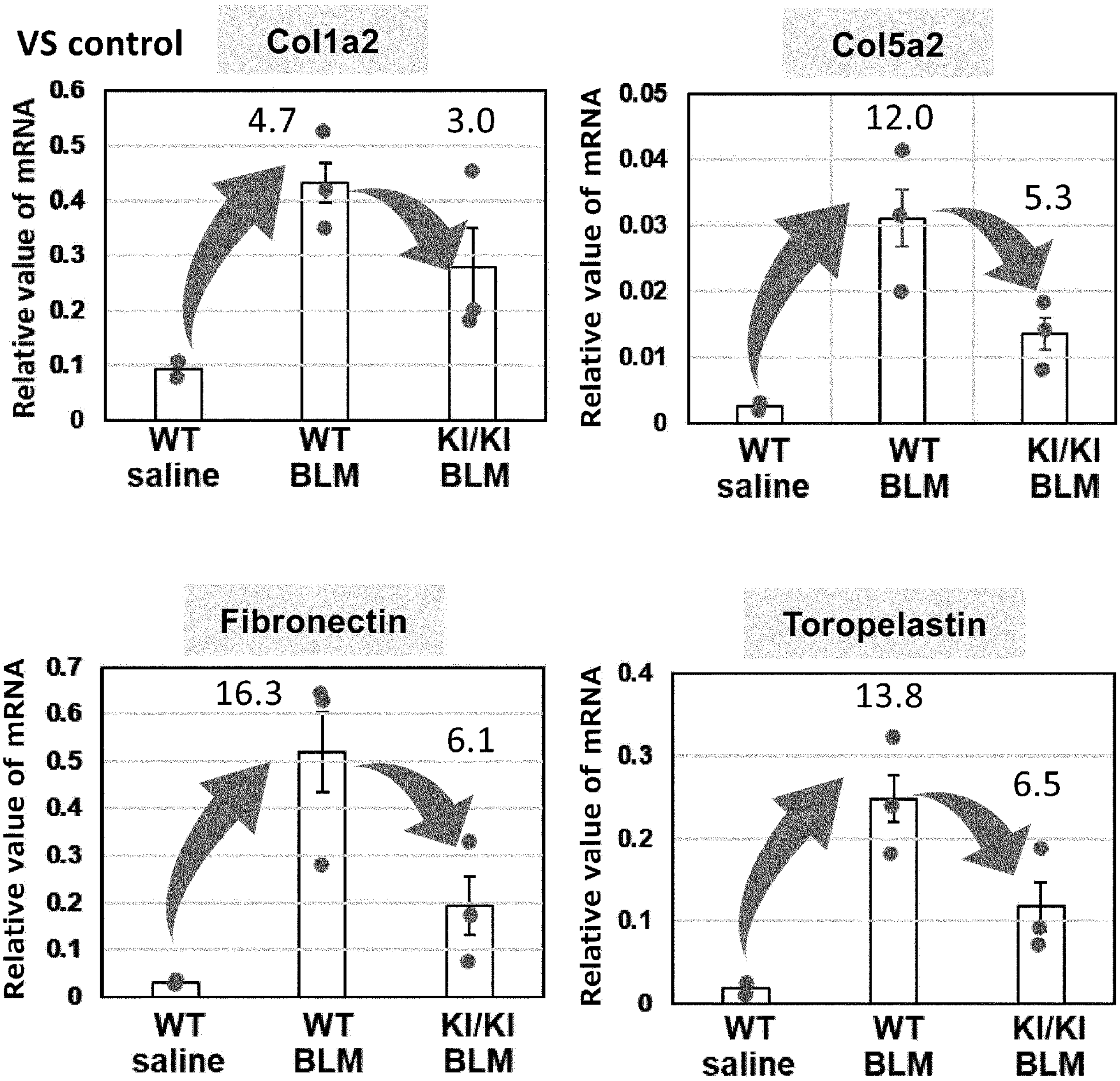
VS control
Col1a2
Relative value of mRNA
0.6
0.5
0.4
0.3
0.2
0.1
0
4.7
3.0
WT saline
WT BLM
KI/KI BLM
Col5a2
Relative value of mRNA
0.05
0.04
0.03
0.02
0.01
0
12.0
5.3
WT saline
WT BLM
KI/KI BLM
Fibronectin
Relative value of mRNA
0.7
0.6
0.5
0.4
0.3
0.2
0.1
0
16.3
6.1
WT saline
WT BLM
KI/KI BLM
Toropelastin
Relative value of mRNA
0.4
0.3
0.2
0.1
0
13.8
6.5
WT saline
WT BLM
KI/KI BLM

[Fig. 10]

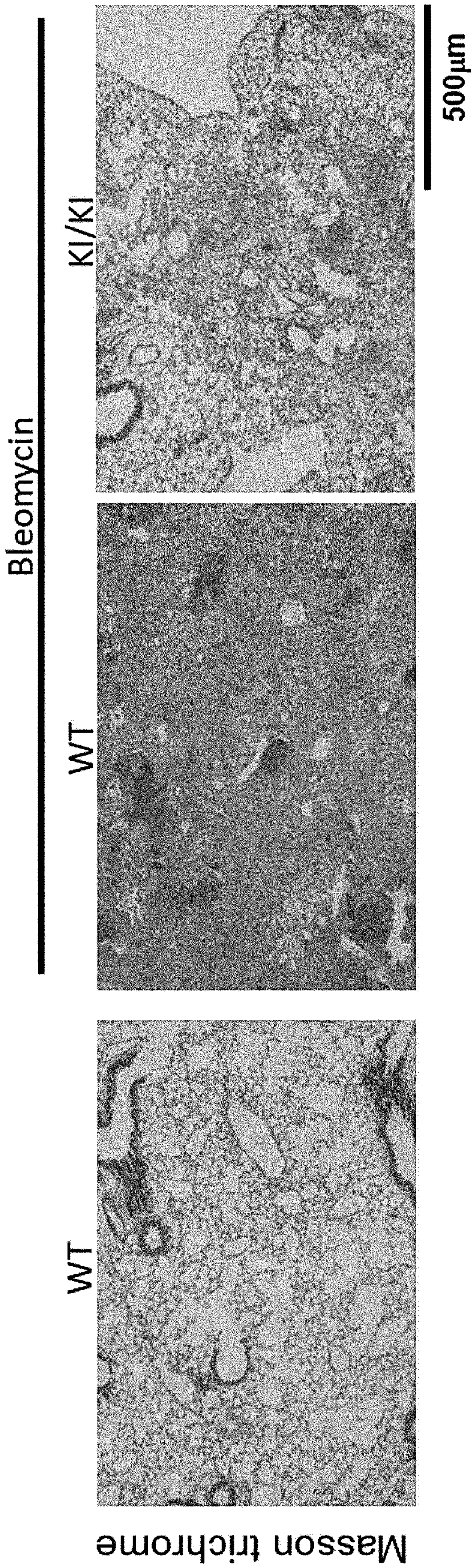

[Fig. 11]

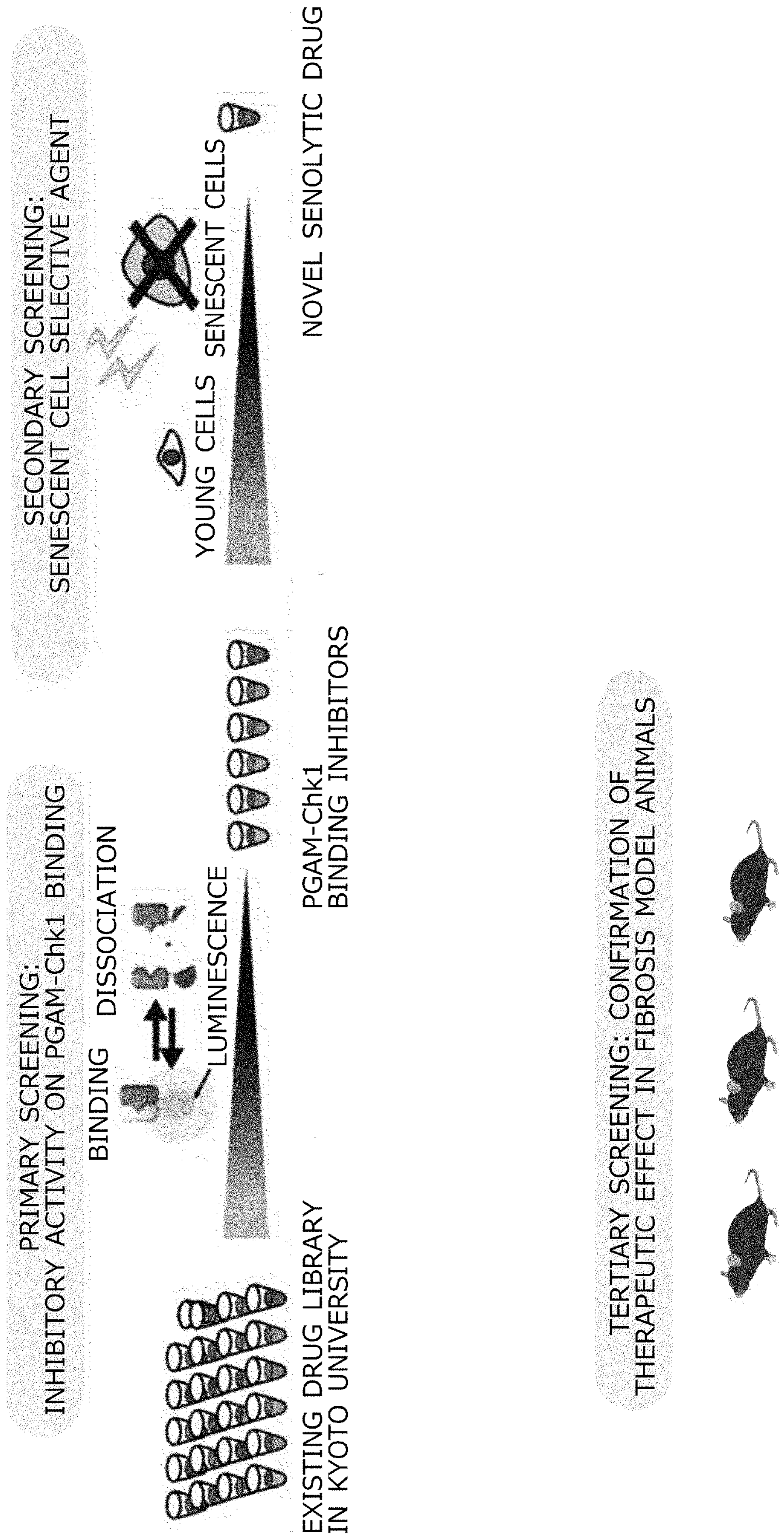
PRIMARY SCREENING:
INHIBITORY ACTIVITY ON PGAM-Chk1 BINDING
BINDING
DISSOCIATION
LUMINESCENCE
EXISTING DRUG LIBRARY
IN KYOTO UNIVERSITY
PGAM-Chk1
BINDING INHIBITORS
SECONDARY SCREENING:
SENESCENT CELL SELECTIVE AGENT
YOUNG CELLS
SENESCENT CELLS
NOVEL SENOLYTIC DRUG
TERTIARY SCREENING: CONFIRMATION OF
THERAPEUTIC EFFECT IN FIBROSIS MODEL ANIMALS

[Fig. 12]

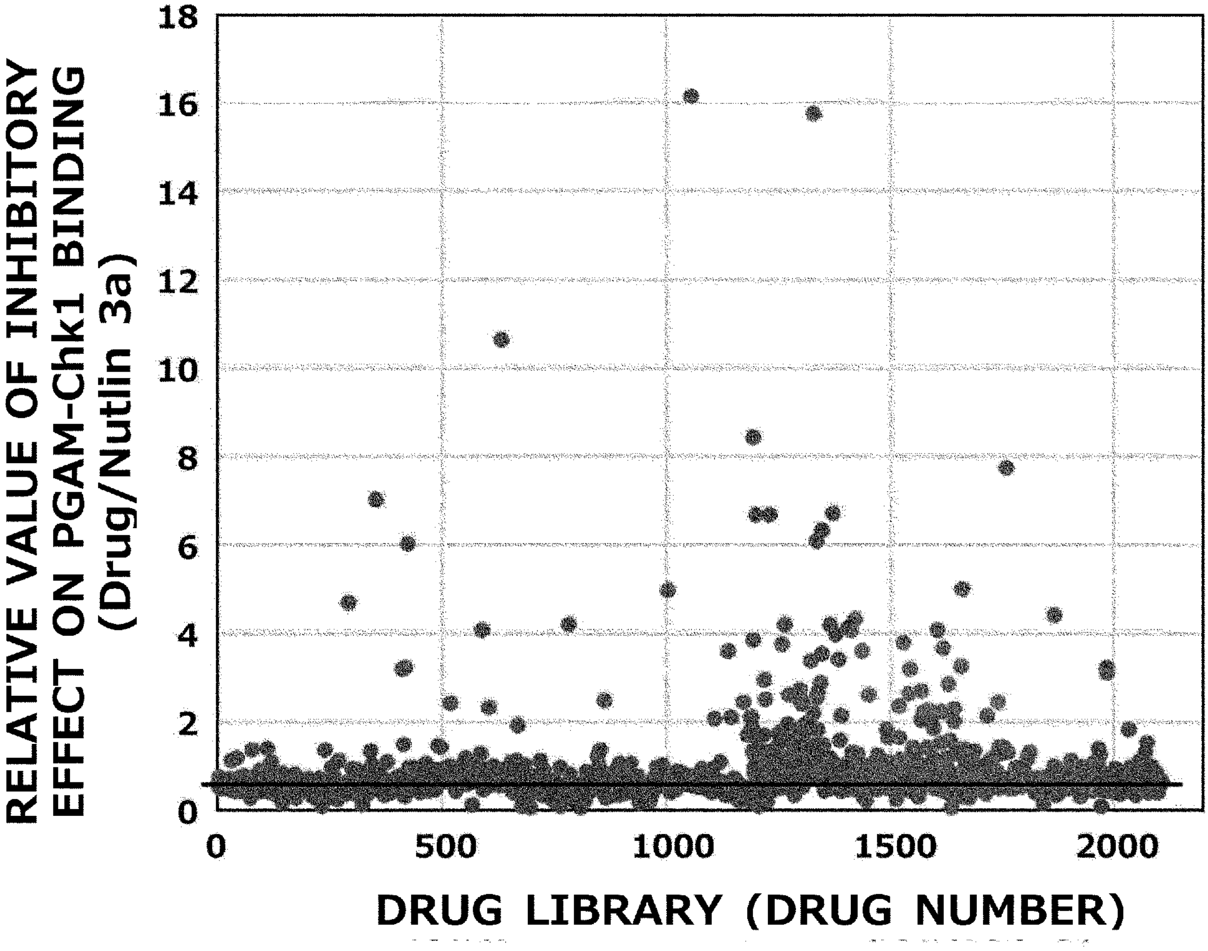
RELATIVE VALUE OF INHIBITORY EFFECT ON PGAM-Chk1 BINDING (Drug/Nutlin 3a)
18
16
14
12
10
8
6
4
2
0
0
500
1000
1500
2000
DRUG LIBRARY (DRUG NUMBER)

[Fig. 13-1]

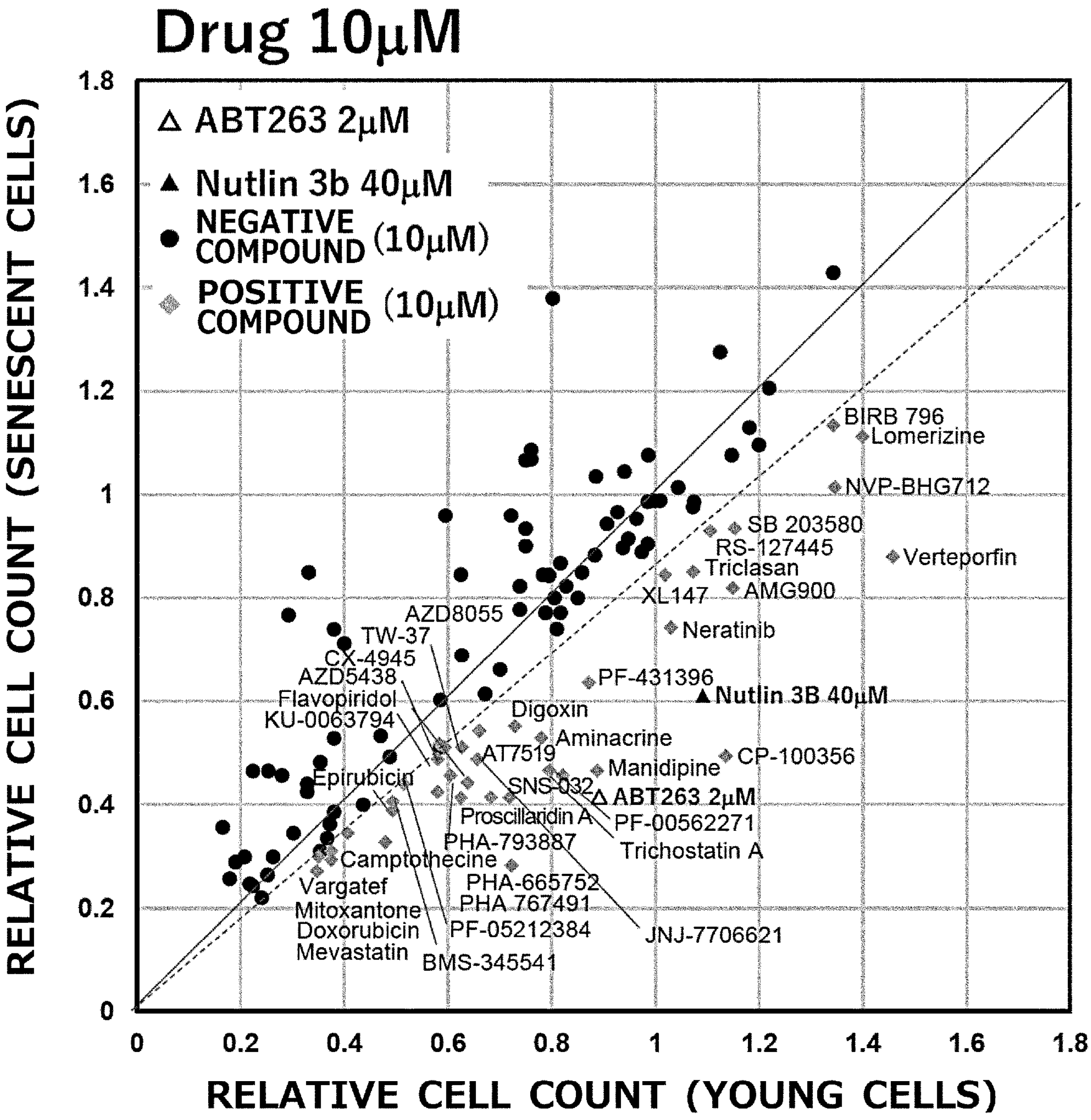
Drug 10μM
ABT263 2μM
Nutlin 3b 40μM
NEGATIVE COMPOUND (10μM)
POSITIVE COMPOUND (10μM)
RELATIVE CELL COUNT (SENESCENT CELLS)
RELATIVE CELL COUNT (YOUNG CELLS)
0
0.2
0.4
0.6
0.8
1
1.2
1.4
1.6
1.8
BIRB 796
Lomerizine
NVP-BHG712
SB 203580
RS-127445
Verteporfin
Triclasan
XL147
AMG900
Neratinib
AZD8055
TW-37
CX-4945
AZD5438
Flavopiridol
KU-0063794
PF-431396
Nutlin 3B 40μM
Digoxin
Aminacrine
AT7519
Manidipine
CP-100356
Epirubicin
SNS-032
ABT263 2μM
Proscillaridin A
PF-00562271
PHA-793887
Trichostatin A
Camptothecine
Vargatef
PHA-665752
Mitoxantone
PHA 767491
Doxorubicin
PF-05212384
JNJ-7706621
Mevastatin
BMS-345541

[Fig. 13-2]

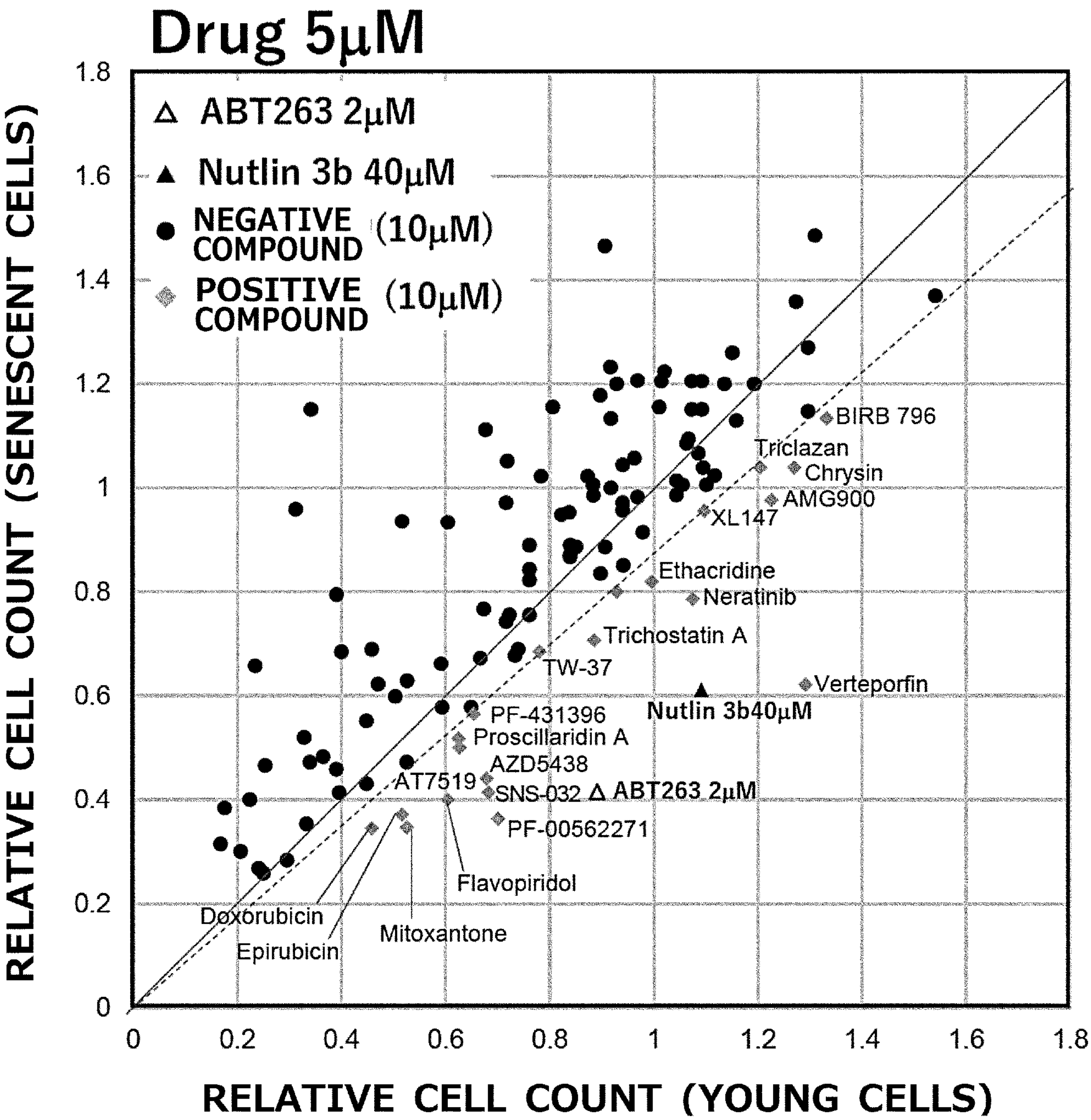
Drug 5μM
ABT263 2μM
Nutlin 3b 40μM
NEGATIVE COMPOUND (10μM)
POSITIVE COMPOUND (10μM)
RELATIVE CELL COUNT (SENESCENT CELLS)
RELATIVE CELL COUNT (YOUNG CELLS)
0
0.2
0.4
0.6
0.8
1
1.2
1.4
1.6
1.8
BIRB 796
Triclazan
Chrysin
AMG900
XL147
Ethacridine
Neratinib
Trichostatin A
TW-37
Verteporfin
Nutlin 3b40μM
PF-431396
Proscillaridin A
AZD5438
AT7519
SNS-032
ABT263 2μM
PF-00562271
Flavopiridol
Doxorubicin
Epirubicin
Mitoxantone

[Fig. 14]

## DRUG CANDIDATES FOR THERAPEUTIC AGENT

Nutlin 3b

RSK90 INHIBITOR
BI D1870

### SCREENING HIT COMPOUNDS (41)

Group 1. FAK,Mdr,cMET INHIBITORS (4)

PHA-665752 (2.56)
CP-100356 (2.38)
PF-00562271 (1.93)
PF-431396 (1.37)

Group 2. CDK INHIBITORS (6)

SNS-032 (1.65)
AZD5438 (1.54)
Flavopiridol (1.51)
PHA-793887 (1.32)
AT7519 (1.25)
PHA-767491 (1.14)

Group 3. CALCIUM ANTAGONISTS (2)

Manidipine (1.91)
Lomerizine (1.26)

Group 4. CARDIAC GLYCOSIDES (2)

Proscillaridin A (1.51)
Digoxin (1.32)

Group 5. DNA-DAMAGINGDRUGS (5)

Doxorubicin (1.46)
Daunorubicin (1.33)
Epirubicin (1.39)
Mitoxantone (1.52)
Camptothecine (1.18)

Group 6. ANTIMICROBIAL DRUGS (3)

Triclazan (1.26)
Aminacrine (1.47)
Ethacridine (1.22)

Group 7. Aurora kinase INHIBITION (2)

JNJ-7706621 (1.35)
AMG900 (1.40)

OTHERS

FLAVONE OR PI3K INHIBITION (3)

Chrysin (1.22)
PF-05212384 (1.17)
XL147 (1.21)

HDAC INHIBITION (1)

Trichostatin A (1.70)

THERAPEUTIC DRUG FOR AGE-RELATED MACULA LUTEA (1)

Verteporfin (2.08)

p38 KINASE INHIBITION (1)

SB203580 (1.24)
BIRB 796 (1.19)

TOR INHIBITORS (2)

AZD8055 (1.23)
KU-0063794 (1.16)

TYROSINE KINASE INHIBITION (3)

Neratinib (1.39)
Vargatef (1.28)
NVP-BHG712 (1.33)

Bcl-2 INHIBITION (1)

TW-37 (1.14)

STATIN (1)

Mevastatin (1.21)

SEROTONIN RECEPTOR ANTAGONIST (1)

RS-127445 (1.21)

OTHER KINASE INHIBITION (2)

BMS-345541 (1.22)
CX-4945 (1.19)

[Fig. 15]

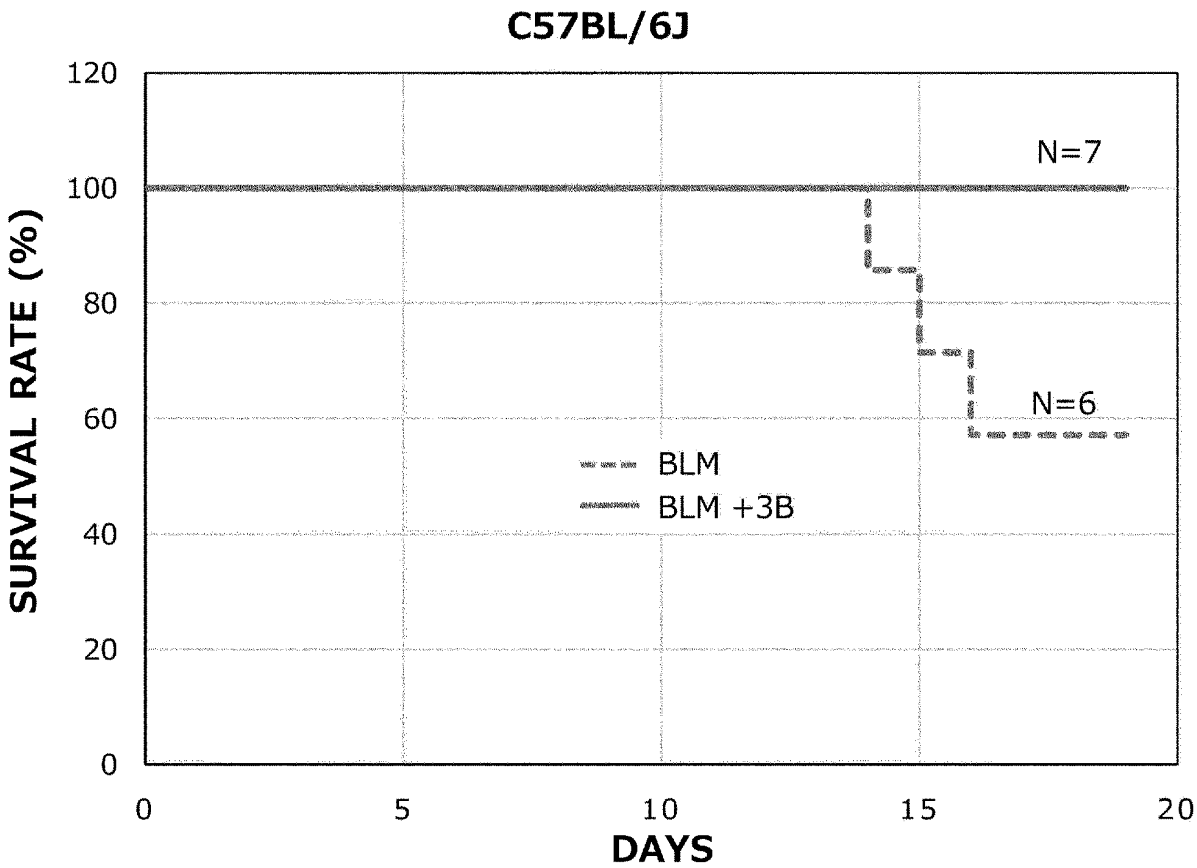


[Fig. 16-1]

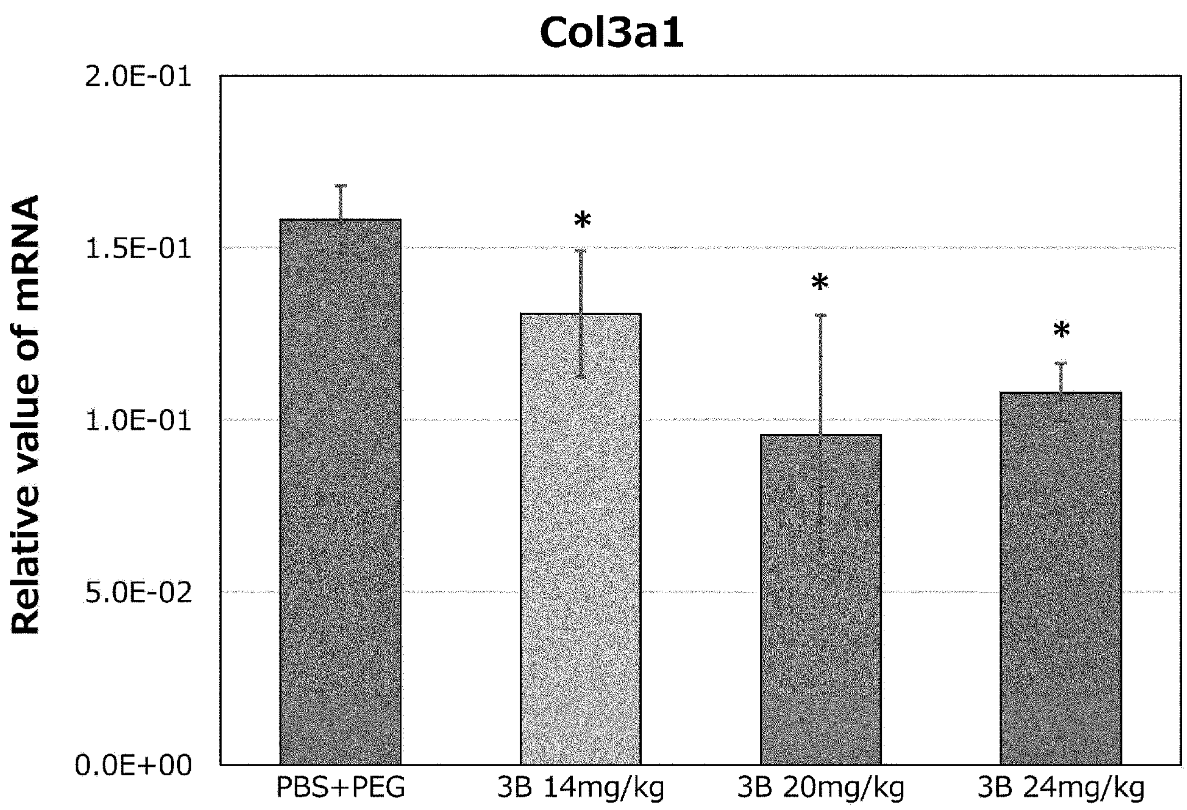

[Fig. 16-2]

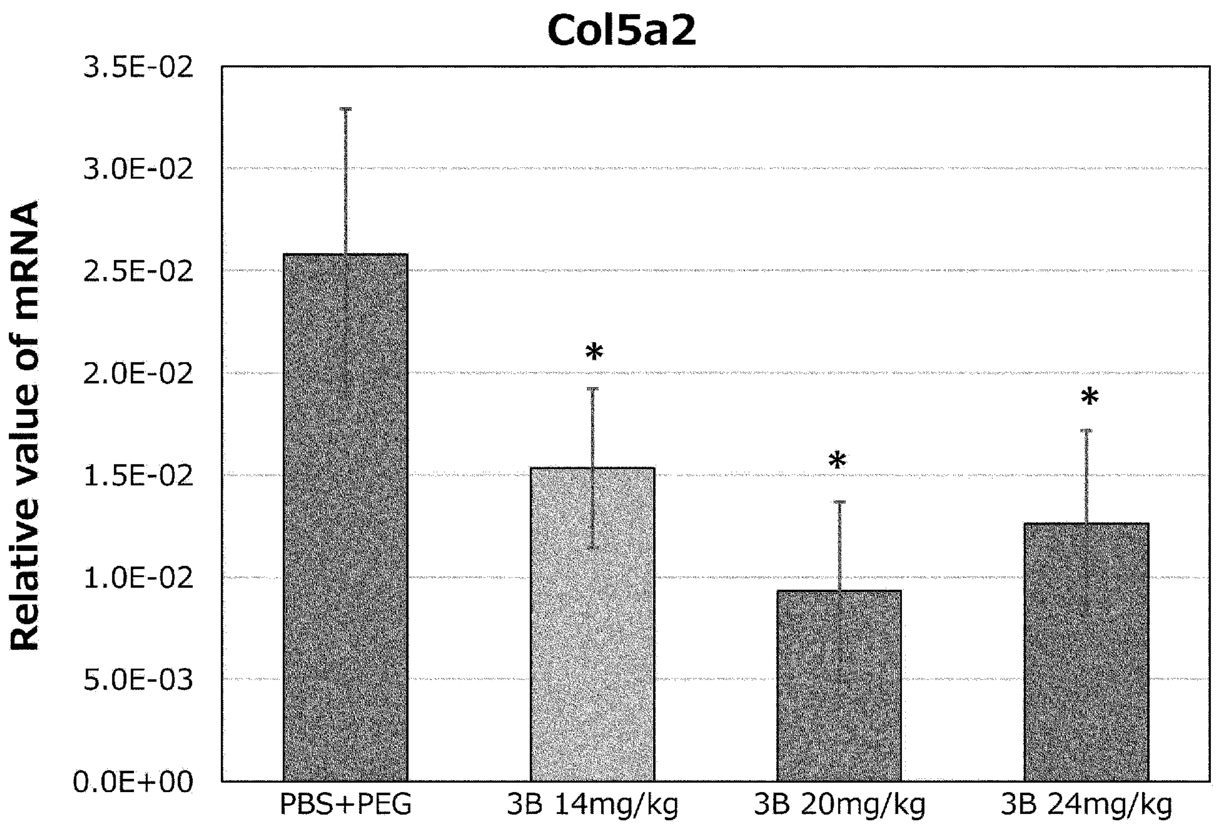


[Fig. 16-3]

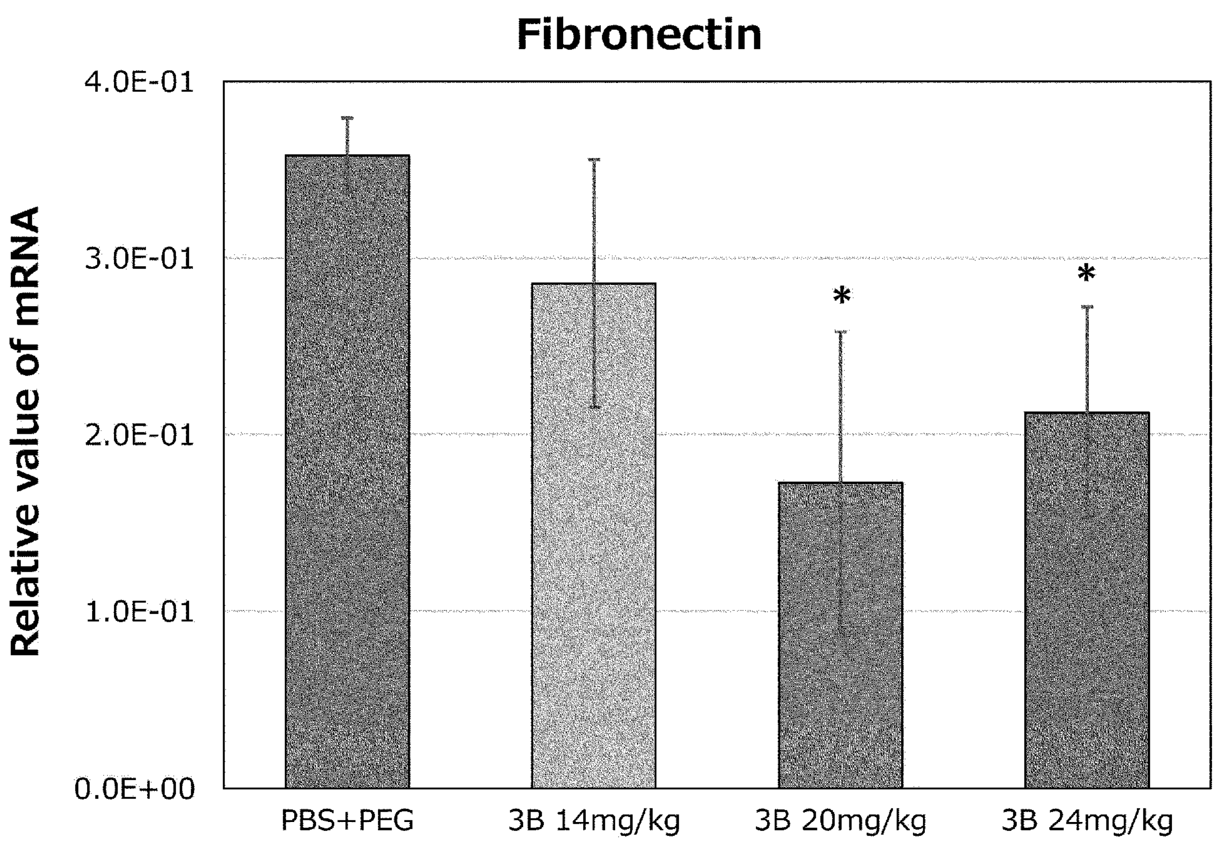

[Fig. 16-4]

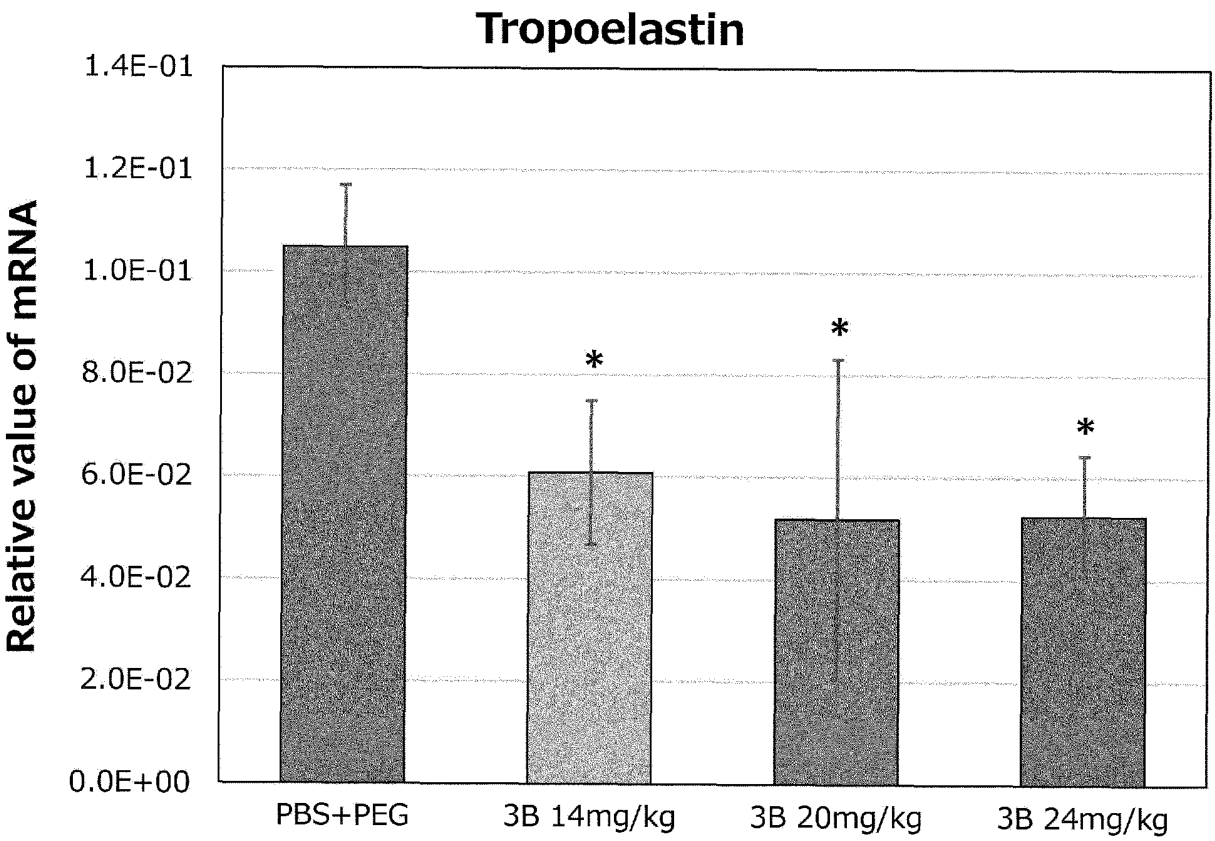
Tropoelastin
Relative value of mRNA
1.4E-01
1.2E-01
1.0E-01
8.0E-02
6.0E-02
4.0E-02
2.0E-02
0.0E+00
*
*
*
PBS+PEG
3B 14mg/kg
3B 20mg/kg
3B 24mg/kg

[Fig. 17-1]

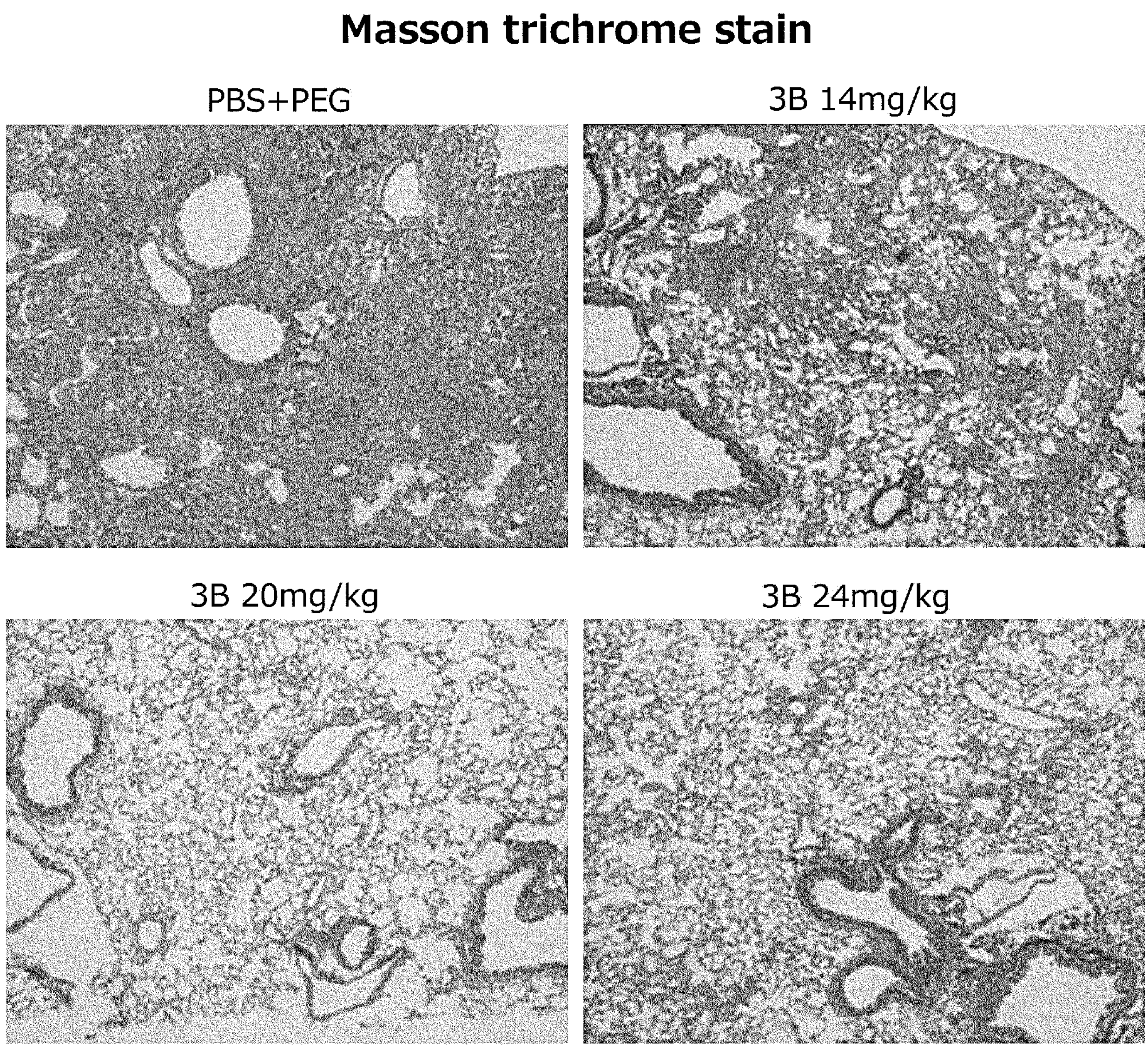
Masson trichrome stain
PBS+PEG
3B 14mg/kg
3B 20mg/kg
3B 24mg/kg

[Fig. 17-2]

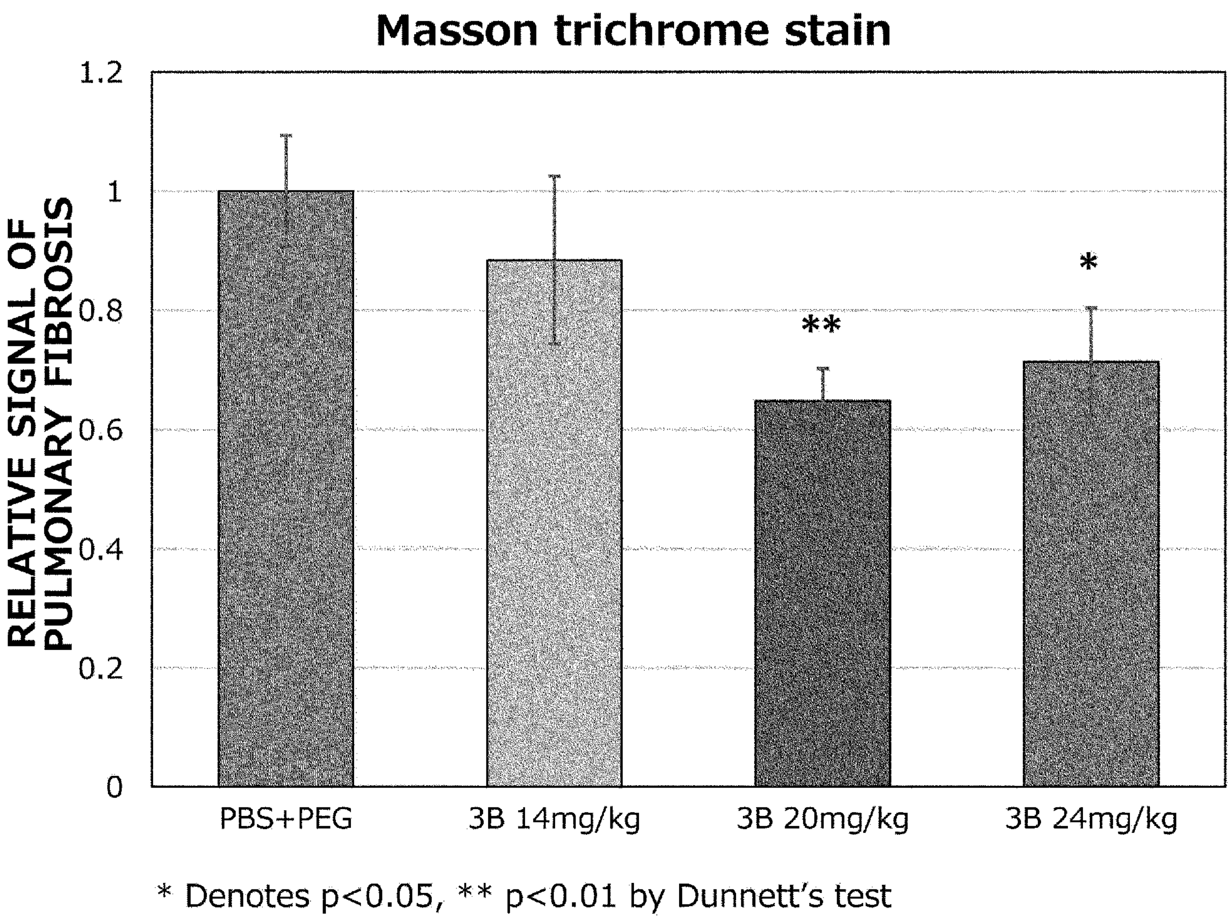
Masson trichrome stain
RELATIVE SIGNAL OF PULMONARY FIBROSIS
1.2
1
0.8
0.6
0.4
0.2
0
**
*
PBS+PEG
3B 14mg/kg
3B 20mg/kg
3B 24mg/kg
* Denotes p<0.05, ** p<0.01 by Dunnett's test

[Fig. 18]

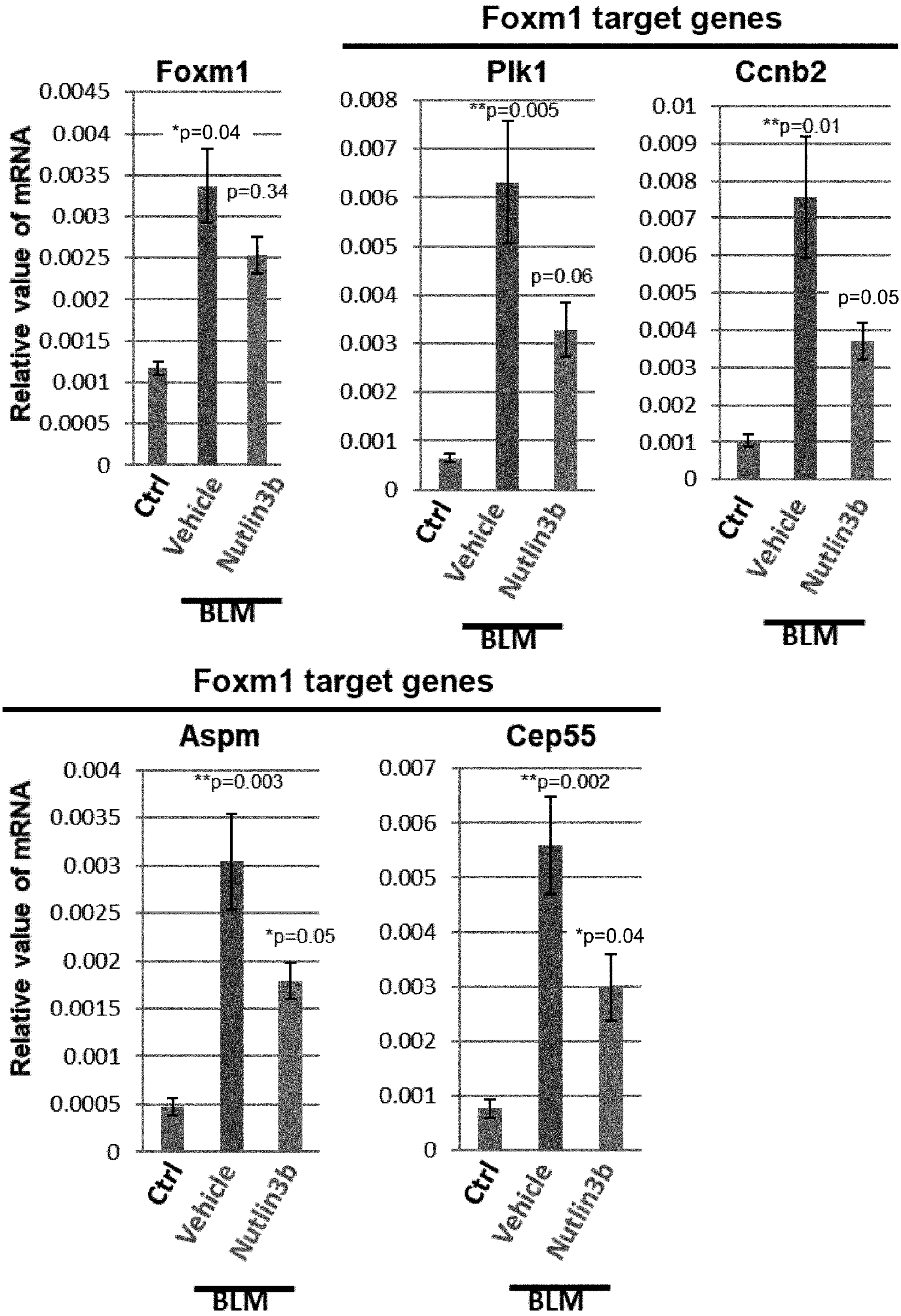
Foxm1 target genes
Foxm1
Plk1
Ccnb2
Relative value of mRNA
0.0045
0.004
0.0035
0.003
0.0025
0.002
0.0015
0.001
0.0005
0
*p=0.04
p=0.34
Ctrl
Vehicle
Nutlin3b
BLM
0.008
0.007
0.006
0.005
0.004
0.003
0.002
0.001
0
**p=0.005
p=0.06
Ctrl
Vehicle
Nutlin3b
BLM
0.01
0.009
0.008
0.007
0.006
0.005
0.004
0.003
0.002
0.001
0
**p=0.01
p=0.05
Ctrl
Vehicle
Nutlin3b
BLM
Foxm1 target genes
Aspm
Cep55
Relative value of mRNA
0.004
0.0035
0.003
0.0025
0.002
0.0015
0.001
0.0005
0
**p=0.003
*p=0.05
Ctrl
Vehicle
Nutlin3b
BLM
0.007
0.006
0.005
0.004
0.003
0.002
0.001
0
**p=0.002
*p=0.04
Ctrl
Vehicle
Nutlin3b
BLM

[Fig. 19-1]

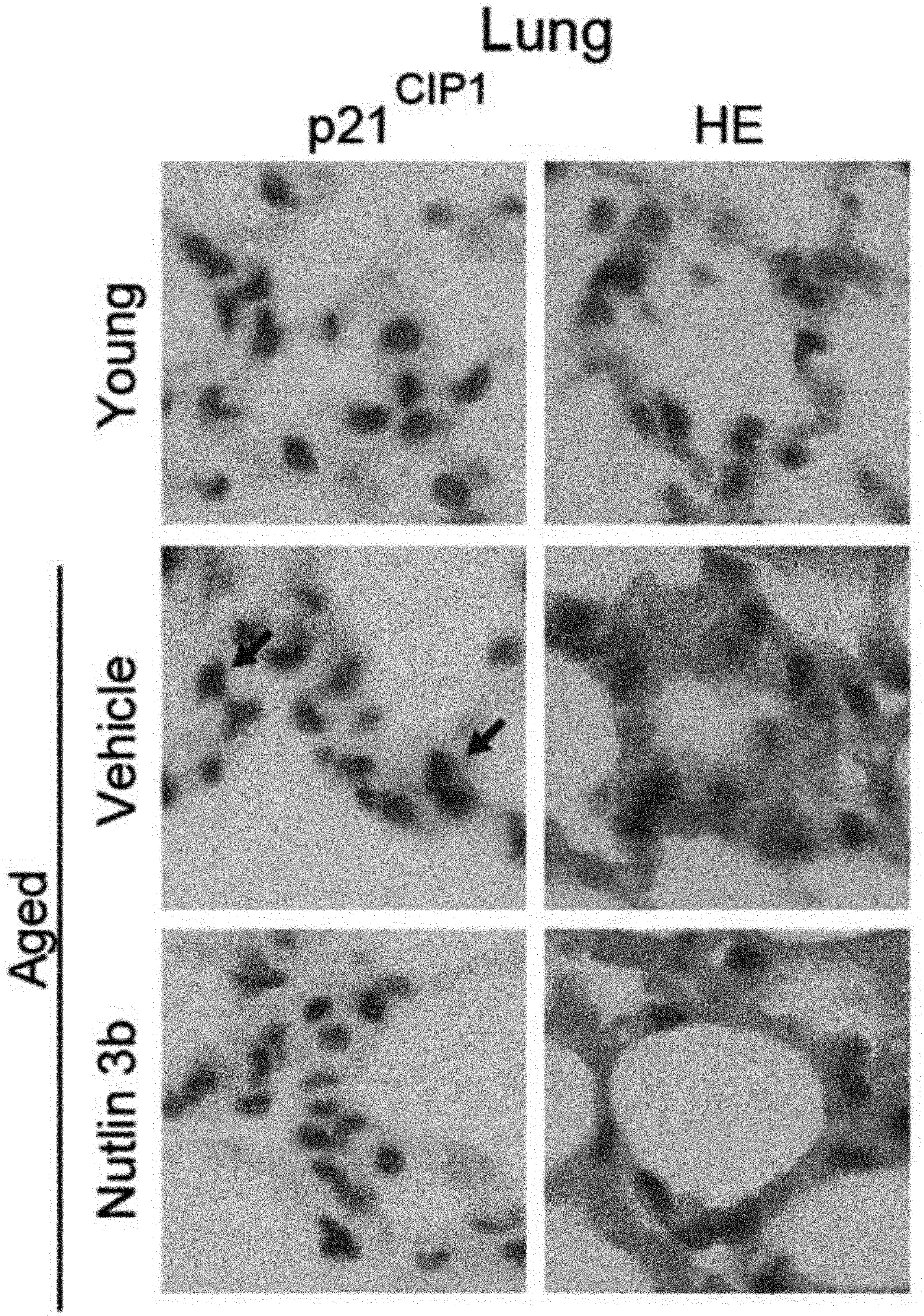

[Fig. 19-2]

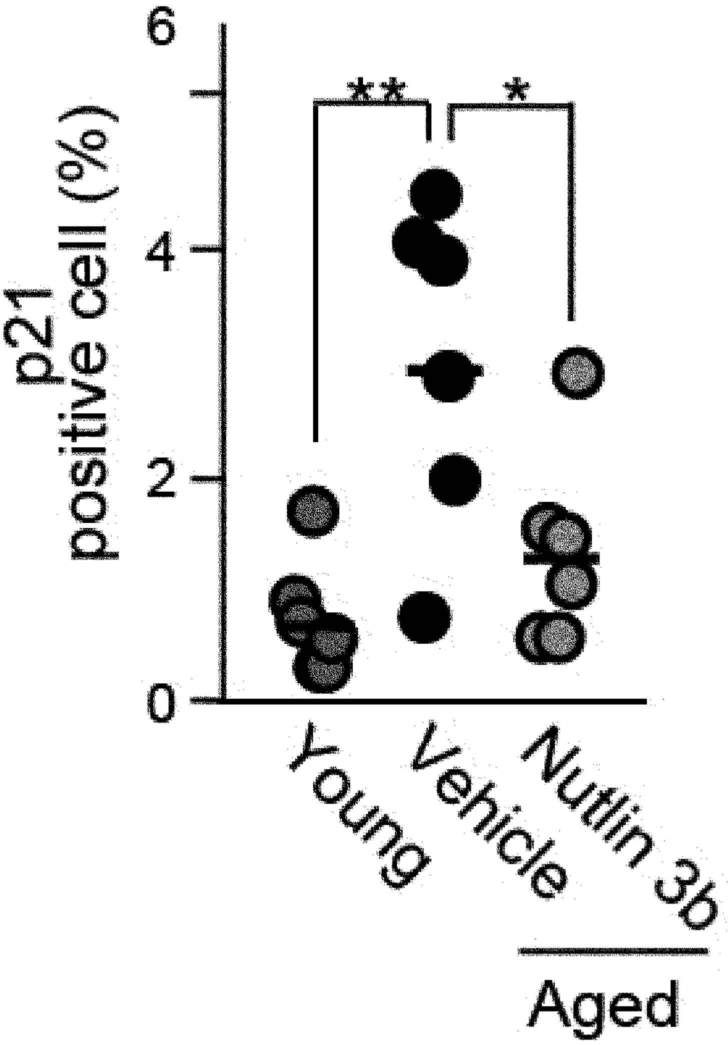

[Fig. 20-1]

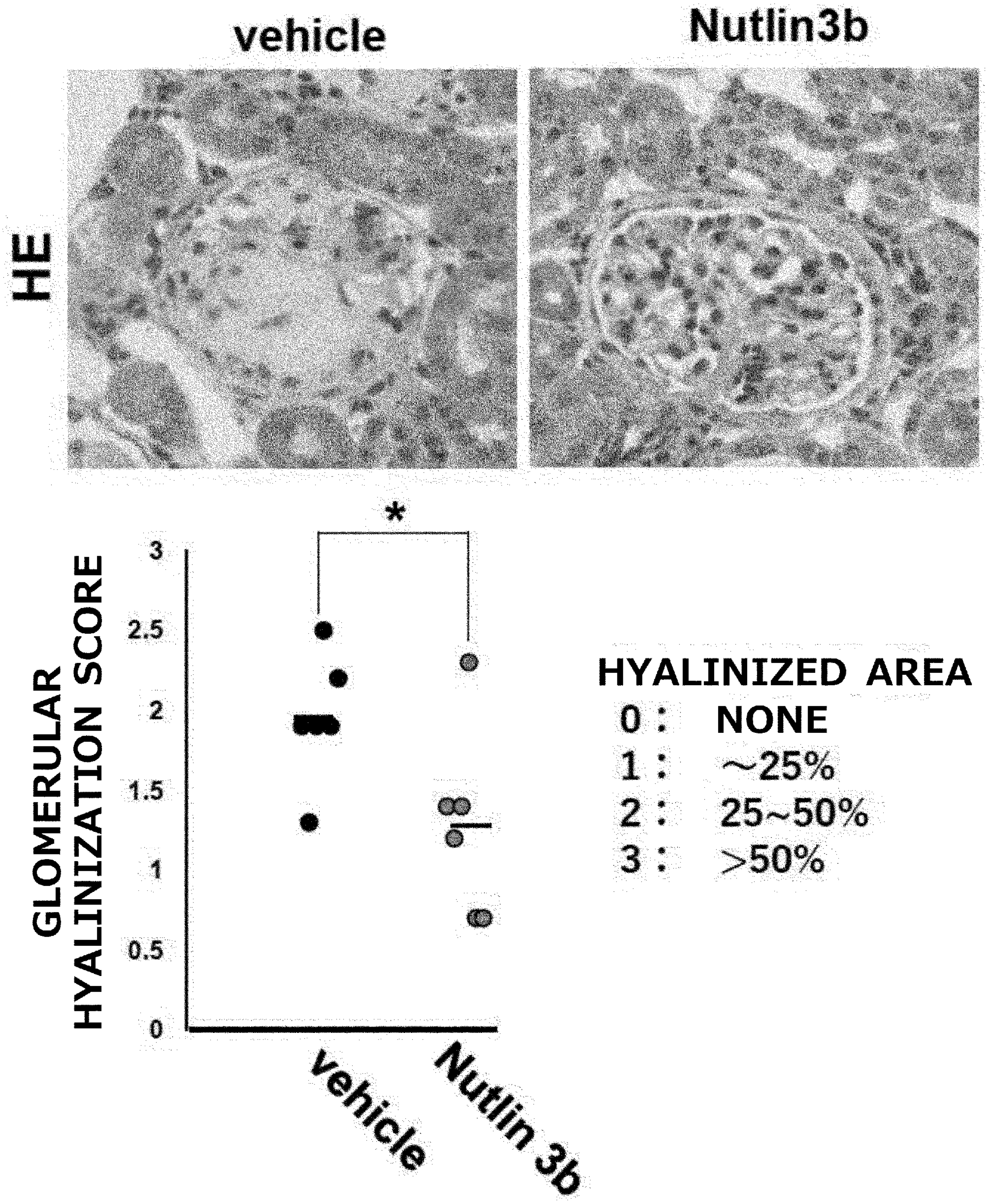
vehicle
Nutlin3b
HE
*
GLOMERULAR HYALINIZATION SCORE
3
2.5
2
1.5
1
0.5
0
vehicle
Nutlin 3b
HYALINIZED AREA
0 : NONE
1 : ~25%
2 : 25~50%
3 : >50%

[Fig. 20-2]

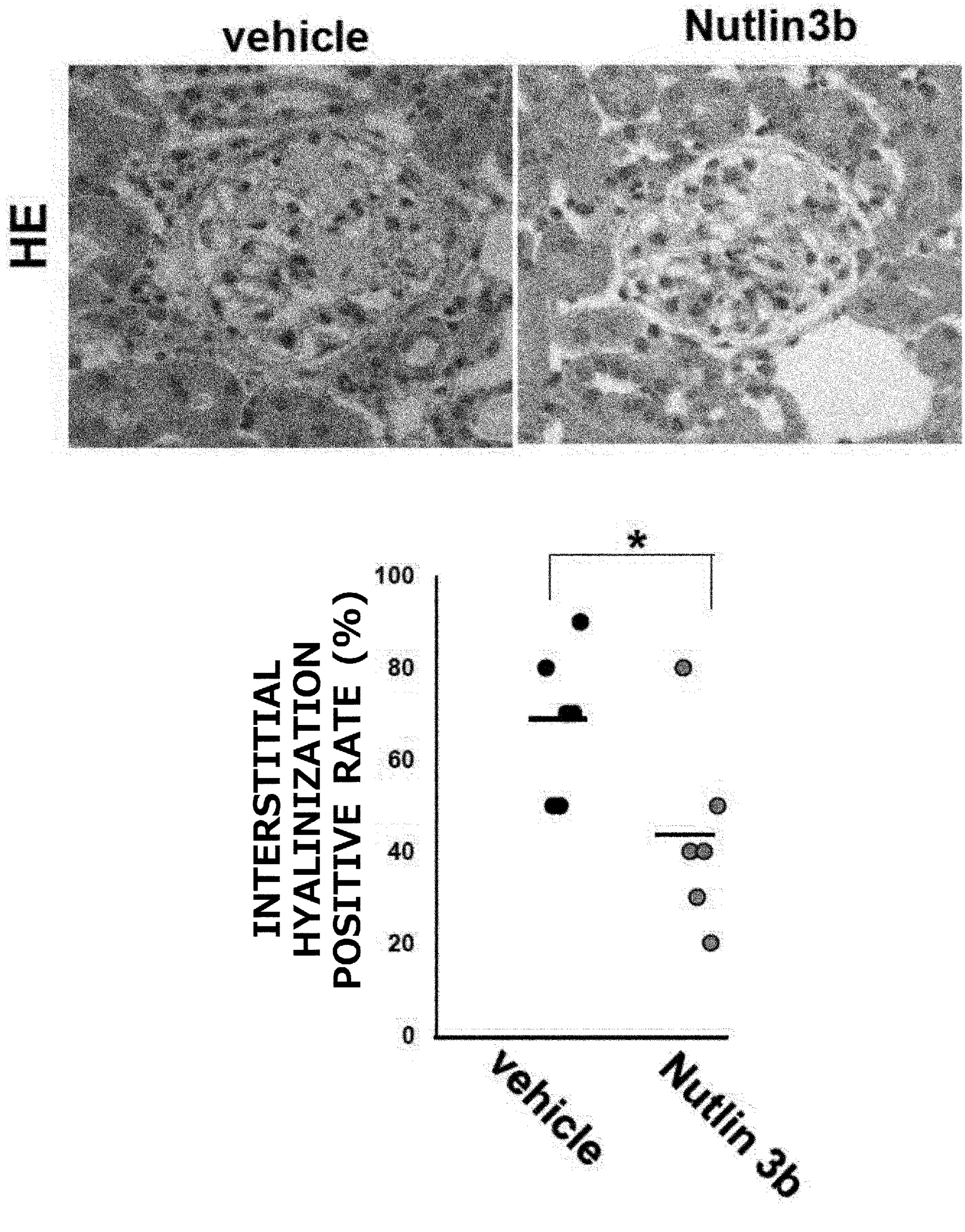
vehicle
Nutlin3b
HE
*
100
80
60
40
20
0
INTERSTITIAL HYALINIZATION POSITIVE RATE (%)
vehicle
Nutlin 3b

[Fig. 20-3]

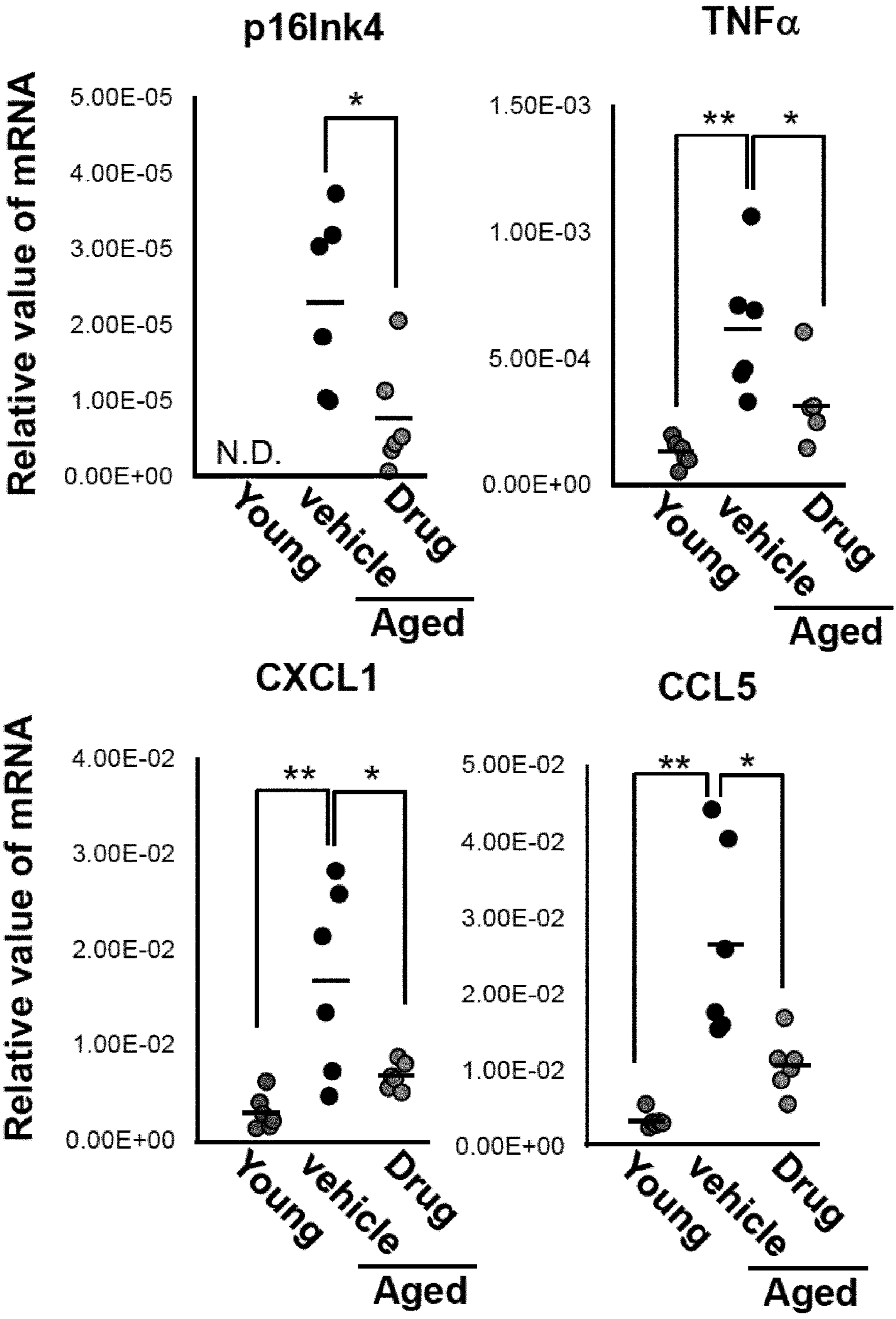


*: p<0.05, **: p<0.01

[Fig. 21]

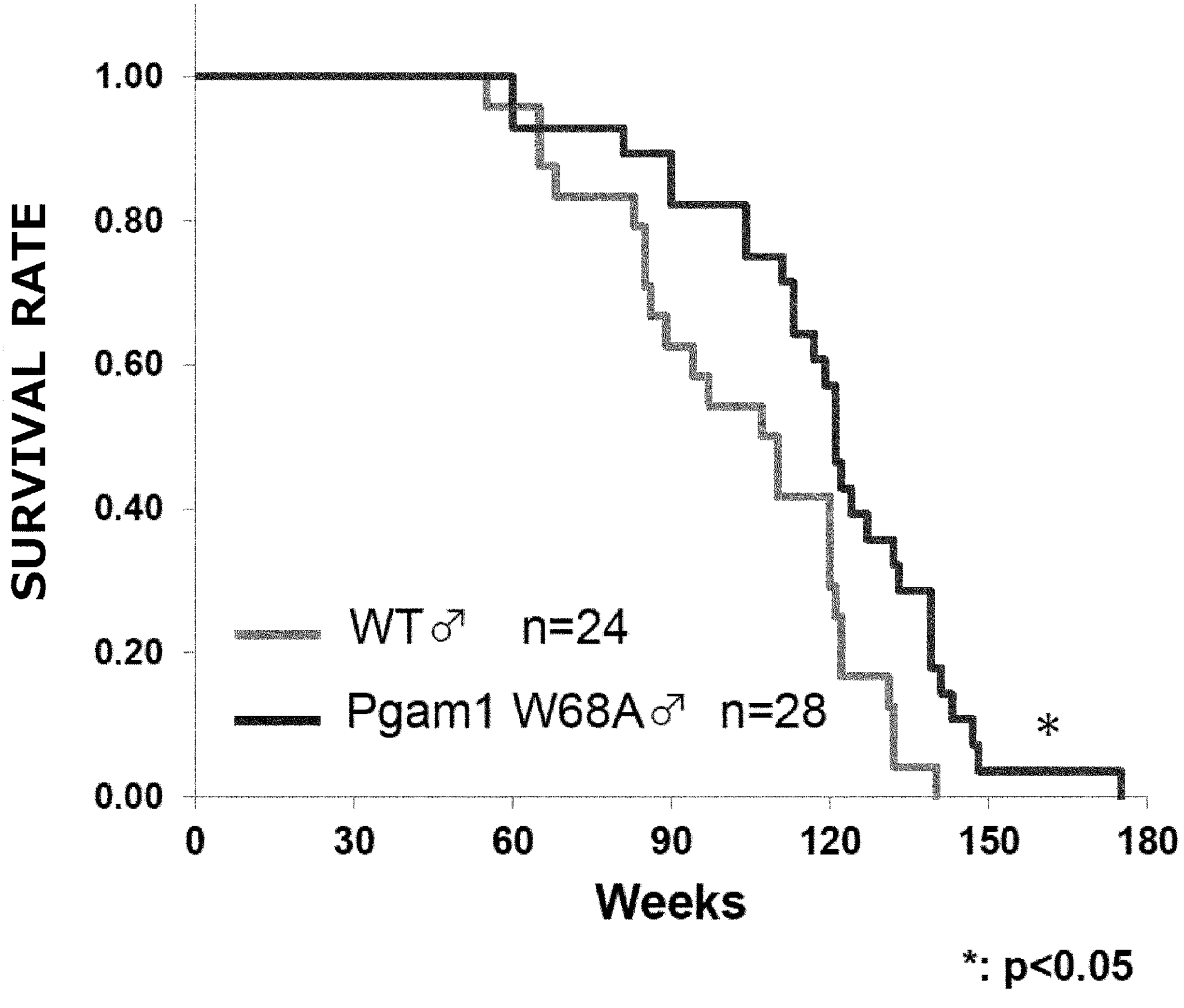
SURVIVAL RATE
1.00
0.80
0.60
0.40
0.20
0.00
0
30
60
90
120
150
180
Weeks
WT♂ n=24
Pgam1 W68A♂ n=28
*
*: p<0.05

**INTERNATIONAL SEARCH REPORT**

International application No. **PCT/JP2023/045647**

**A. CLASSIFICATION OF SUBJECT MATTER**

***A61K 45/00***(2006.01)i; ***A01K 67/0278***(2024.01)i; ***A61K 31/085***(2006.01)i; ***A61K 31/136***(2006.01)i; ***A61K 31/165***(2006.01)i; ***A61K 31/166***(2006.01)i; ***A61K 31/351***(2006.01)i; ***A61K 31/352***(2006.01)i; ***A61K 31/403***(2006.01)i; ***A61K 31/409***(2006.01)i; ***A61K 31/444***(2006.01)i; ***A61K 31/453***(2006.01)i; ***A61K 31/454***(2006.01)i; ***A61K 31/473***(2006.01)i; ***A61K 31/495***(2006.01)i; ***A61K 31/496***(2006.01)i; ***A61K 31/498***(2006.01)i; ***A61K 31/505***(2006.01)i; ***A61K 31/506***(2006.01)i; ***A61K 31/517***(2006.01)i; ***A61K 31/519***(2006.01)i; ***A61K 31/635***(2006.01)i; ***A61K 31/704***(2006.01)i; ***A61K 31/713***(2006.01)i; ***A61K 31/4439***(2006.01)i; ***A61K 31/4709***(2006.01)i; ***A61K 31/4745***(2006.01)i; ***A61K 31/4985***(2006.01)i; ***A61K 31/5377***(2006.01)i; ***A61K 31/7048***(2006.01)i; ***A61K 31/7088***(2006.01)i; ***A61K 48/00***(2006.01)i; ***A61P 1/16***(2006.01)i; ***A61P 11/00***(2006.01)i; ***A61P 43/00***(2006.01)i

FI: A61K45/00 ZNA; A01K67/0278; A61K31/085; A61K31/136; A61K31/165; A61K31/166; A61K31/351; A61K31/352; A61K31/403; A61K31/409; A61K31/4439; A61K31/444; A61K31/453; A61K31/454; A61K31/4709; A61K31/473; A61K31/4745; A61K31/495; A61K31/496; A61K31/498; A61K31/4985; A61K31/505; A61K31/506; A61K31/517; A61K31/519; A61K31/5377; A61K31/635; A61K31/704; A61K31/7048; A61K31/7088; A61K31/713; A61K48/00; A61P1/16; A61P11/00; A61P43/00 111

According to International Patent Classification (IPC) or to both national classification and IPC

**B. FIELDS SEARCHED**

Minimum documentation searched (classification system followed by classification symbols)

A61K45/00; A01K67/0278; A61K31/085; A61K31/136; A61K31/165; A61K31/166; A61K31/351; A61K31/352; A61K31/403; A61K31/409; A61K31/444; A61K31/453; A61K31/454; A61K31/473; A61K31/495; A61K31/496; A61K31/498; A61K31/505; A61K31/506; A61K31/517; A61K31/519; A61K31/635; A61K31/704; A61K31/713; A61K31/4439; A61K31/4709; A61K31/4745; A61K31/4985; A61K31/5377; A61K31/7048; A61K31/7088; A61K48/00; A61P1/16; A61P11/00; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); PubMed; 医中誌Web

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 March 2024** | **02 April 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2023/045647**

**C. DOCUMENTS CONSIDERED TO BE RELEVANT**

| Category* | Citation of document, with indication, where appropriate, of the relevant passages | Relevant to claim No. |
|---|---|---|
| X | CHO, H. et al., Nintedanib induces senolytic effect via STAT3 inhibition, Cell Death & Disease, 02 September 2022, vol. 13, no. 9, 760, doi:10.1038/s41419-022-05207-8, ISSN 2041-4889<br>particularly, abstract, page 1, left column, second paragraph to right column, line 1, page 2, left column, first paragraph, page 7, 9-10, Nintedanib reduces the number of SAβG-positive senescent cells in bleomycin-induced lung fibrosis, fig. 5 | 1-6, 9 |
| Y | page 1, left column, second paragraph to right column, line 1 | 9 |
| X | TRIANA-MARTINEZ, F. et al., Identification and characterization of Cardiac Glycosides as senolytic compounds, Nature Communications, 2019, vol. 10, no. 1, 4731, doi:10.1038/s41467-019-12888-x, ISSN 2041-1723<br>particularly, page 1, abstract, pages 8-9, Digoxin is senolytic in lung fibrosis, page 10, right column, lines 30-34, fig. 6 | 1-6 |
| Y | particularly, page 1, abstract, pages 8-9, Digoxin is senolytic in lung fibrosis, page 10, right column, lines 30-34, fig. 6 | 9 |
| Y | MIKAWA, T. et al., Phosphoglycerate Mutase Cooperates with Chk1 Kinase to Regulate Glycolysis, iScience, 2020, vol. 23, no. 7, 101306, doi:10.1016/j.isci.2020.101306, ISSN 2589-0042<br>particularly, page 10, Nutlin-3a Interferes with the PGAM-Chk1 Interaction, fig. 6 | 7-8 |
| Y | 岡田季之 ほか, 遺伝子操作マウスを使った研究のピットフォール, 消化器病学サイエンス, 2021, vol. 5, no. 2, pages 95-98, ISSN 2432-7549, (Science of Gastroenterology), non-official translation (OKADA, Toshiyuki et al., Pitfall of research using genetically engineered mice)<br>particularly, page 97, 5. ノックインマウス(KIマウス), non-official translation (Knock-in mouse (KI mouse)) | 7-8 |
| A | JP 2018-194299 A (KYOTO UNIVERSITY) 06 December 2018 (2018-12-06)<br>claims, examples | 1-9 |
| A | 荒谷紗絵, 中西真, 老化細胞除去による腎老化治療, アンチ・エイジング医学, 2021, vol. 17, no. 4, pages 318-323, ISSN 1880-1579, (ARATANI, Sae, NAKANISHI, Makoto, Senolytics for kidney aging, Anti-Aging Medicine)<br>entire text, all drawings | 1-9 |
| A | 杉本昌隆, 三河隆太, 老化細胞除去マウスとsenolytic薬, 実験医学, 2019, vol. 37, no. 11, pages 1755-1760, ISSN 0288-5514, (SUGIMOTO, Masataka, MIKAWA, Ryuta, Experimental Medicine), non-official translation (Senenolytic mice and senolytic drugs)<br>entire text, all drawings | 1-9 |
| P, A | WO 2023/038027 A1 (KYOTO UNIVERSITY) 16 March 2023 (2023-03-16)<br>claims, examples | 1-9 |

Form PCT/ISA/210 (second sheet) (July 2022)

**INTERNATIONAL SEARCH REPORT**

International application No.
**PCT/JP2023/045647**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2023/045647**

| Patent document cited in search report | Publication date (day/month/year) | Patent family member(s) | Publication date (day/month/year) |
|---|---|---|---|
| JP 2018-194299 A | 06 December 2018 | (Family: none) | |
| WO 2023/038027 A1 | 16 March 2023 | TW 202327606 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018194299 A


### Non-patent literature cited in the description


- *Pharmacol Ther*, 03 May 2015
- *Mutation Research*, 2012, vol. 730 (1-2), 52-58
- *CHEMICAL ABSTRACTS*, 501437-28-1
- *CHEMICAL ABSTRACTS*, 939791-41-0
- *CHEMICAL ABSTRACTS*, 717906-29-1
- *CHEMICAL ABSTRACTS*, 477575-56-7
- *CHEMICAL ABSTRACTS*, 142715-48-8
- *CHEMICAL ABSTRACTS*, 345627-80-7
- *CHEMICAL ABSTRACTS*, 602306-29-6
- *CHEMICAL ABSTRACTS*, 146426-40-6
- *CHEMICAL ABSTRACTS*, 718630-59-2
- *CHEMICAL ABSTRACTS*, 844442-38-2
- *CHEMICAL ABSTRACTS*, 942425-68-5
- *CHEMICAL ABSTRACTS*, 89226-50-6
- *CHEMICAL ABSTRACTS*, 101477-54-7
- *CHEMICAL ABSTRACTS*, 466-06-8
- *CHEMICAL ABSTRACTS*, 20830-75-5
- *CHEMICAL ABSTRACTS*, 25316-40-9
- *CHEMICAL ABSTRACTS*, 23541-50-6
- *CHEMICAL ABSTRACTS*, 56390-09-1
- *CHEMICAL ABSTRACTS*, 70476-82-3
- *CHEMICAL ABSTRACTS*, 64439-81-2
- *CHEMICAL ABSTRACTS*, 90-45-9
- *CHEMICAL ABSTRACTS*, 3380-34-5
- *CHEMICAL ABSTRACTS*, 1837-57-6
- *CHEMICAL ABSTRACTS*, 945595-80-2
- *CHEMICAL ABSTRACTS*, 443797-96-4
- **IYER et al.** *Genes and Development*, 1998, vol. 12, 149-62
- *CHEMICAL ABSTRACTS*, 675576-97-3
- *CHEMICAL ABSTRACTS*, 480-40-0
- *CHEMICAL ABSTRACTS*, 1197160-78-3
- *CHEMICAL ABSTRACTS*, 934526-89-3
- *CHEMICAL ABSTRACTS*, 58880-19-6
- *CHEMICAL ABSTRACTS*, 129497-78-5
- *CHEMICAL ABSTRACTS*, 152121-47-6
- *CHEMICAL ABSTRACTS*, 285983-48-4
- *CHEMICAL ABSTRACTS*, 1009298-09-2
- *CHEMICAL ABSTRACTS*, 938440-64-3
- *CHEMICAL ABSTRACTS*, 698387-09-6
- *CHEMICAL ABSTRACTS*, 656247-17-5
- *CHEMICAL ABSTRACTS*, 940310-85-0
- *CHEMICAL ABSTRACTS*, 877877-35-5
- *CHEMICAL ABSTRACTS*, 73573-88-3
- *CHEMICAL ABSTRACTS*, 199864-87-4
- *CHEMICAL ABSTRACTS*, 445430-58-0
- *CHEMICAL ABSTRACTS*, 1009820-21-6